# FASCICULE DE BREVET EUROPEEN

(11) **EP 2 033 663 B1**
(45) Date de publication et mention de la délivrance du brevet: **02.04.2014**
(21) Numéro de dépôt: 08290835.1
(22) Date de dépôt: 05.09.2008
(51) Int. Cl.: A61K 49/00, G01N 33/52, G01N 33/542

(54) **Substrats fluorescents saccharidiques, leur procédé de préparation et leurs utilisations**
Fluoreszierende Saccharidsubstrate, ihr Herstellungsverfahren und ihre Anwendungen
Saccharidic fluorescent substrates, method of preparing same and use of same

(30) Priorité: 10.09.2007 FR 0706313
(43) Date de publication de la demande: 11.03.2009
(73) Titulaire: Commissariat à l'Énergie Atomique et aux Énergies Alternatives, 75015 Paris (FR)
(72) Inventeur: Durrat, François, 38000 Grenoble (FR); Texier-Nogues, Isabelle, 38000 Grenoble (FR); Robert, Véronique, 38000 Grenoble (FR)
(74) Mandataire: Noel, Chantal Odile

(56) Documents cités:
- FR-A- 2 888 938
- US-A1- 2006 147 378
- US-A1- 2007 009 980
- HO NAN-HUI ET AL: "A self-immolative reporter for beta-galactosidase sensing." CHEMBIOCHEM : A EUROPEAN JOURNAL OF CHEMICAL BIOLOGY 26 MAR 2007, vol. 8, no. 5, 26 mars 2007 (2007-03-26), pages 560-566, XP002477908 ISSN: 1439-4227

## Description

La présente invention est relative à des substrats enzymatiques fluorescents de nature saccharidique comportant un bras espaceur autosécable fonctionnalisé par un fluorophore F et par au moins un inhibiteur de la fluorescence de F, à leur utilisation pour la préparation d'un réactif de diagnostic pour l'imagerie fonctionnelle *in vivo,* ainsi qu'au réactif de diagnostic pour l'imagerie fonctionnelle renfermant au moins un tel substrat enzymatique.

La fluorescence est une technique très utilisée pour la détection d'activités enzymatiques *in vitro.* C'est une technique peu coûteuse, rapide et généralement très sensible. De nombreuses enzymes d'importance biologique significative ont pour substrats des dérivés saccharidiques qui peuvent notamment être utilisés pour effectuer des dosages enzymatiques sur des échantillons biologiques (sang, urine, ...), sur des cellules (fixées ou en culture) ou bien encore sur des tissus (tissus d'animaux sacrifiés, biopsies).

Des applications qui semblent également prometteuses sont les applications *in vivo,* notamment pour l'imagerie de fluorescence du petit animal. En effet, des gènes rapporteurs exprimant différentes enzymes telles que la β -galactosidase (β-gal), la β-glucuronidase (β-glu), le chloramphénicol, l'acétyltransférase, la luciférase, les protéines fluorescentes telles que la "Green Fluorescent Protein" (GFP), sont très utilisés aujourd'hui en biologie pour étudier l'expression de gènes (transcription et traduction de l'ADN en protéines), la transfection, ou d'autres processus biologiques. Les gènes rapporteurs peuvent servir de témoins pour démontrer l'introduction et la transcription d'un autre gène d'intérêt situé sur la même partie codante de l'ADN. Les constructions d'ADN contenant les gènes rapporteurs sont introduites dans l'animal pour former des animaux transgéniques. Par exemple, le nombre de souris transgéniques déjà construites est très important et en augmentation rapide. Dans un très grand nombre de cas, le gène marqueur utilisé est le gène /*acZ* qui code pour la β-gal de *E. coli.* Un autre exemple de gène marqueur également utilisé est le gène gusA qui code pour la β-glu de *E. coli.* Or, les substrats des enzymes exprimées par certains de ces gènes, et en particulier par les gènes *lacZ* et *gusA,* sont des dérivés saccharidiques. Il est donc très important de pouvoir disposer de substrats saccharidiques pour pouvoir détecter l'activité de ces enzymes.

De nombreux substrats de nature saccharidique existent déjà pour détecter des activités enzymatiques telles que par exemple les activités enzymatiques de β-gal et β-glu. Ces substrats enzymatiques peuvent notamment être :
- des substrats pour l'imagerie nucléaire,
- des substrats chimiluminescents tels que les substrats commercialisés sous les dénominations commerciales Lumi-Gal ® 530 par la société Lumigen Inc. (USA) et Galacton-*Star*® par la société Applied-Biosystems (USA);
- des substrats pour la détection diélectrophorétique,
- des substrats pour l'IRM,
- des substrats formant des précipités,
- des substrats pour dosages spectrophotométriques dont le substrat X-gal (5-bromo-4-chloro-3-indolyl-beta-D-galactopyranoside) vendu par exemple sous la dénomination commerciale *Blue*Tech ® par la société Mirador DNA Design Inc.; et
- des substrats fluorescents. Idéalement, ces substrats doivent avoir les propriétés suivantes :

- une cinétique de réaction enzymatique rapide,
- une constante de Michaelis faible,
- une grande différence entre les propriétés d'intérêt du substrat et celles de son(ses) produit(s) (propriétés d'intérêt : absorption pour un substrat chromogénique, fluorescence pour un substrat fluorogénique, etc...).

L'intérêt des substrats fluorescents, par rapport aux autres substrats décrits ci-dessus, est leur sensibilité de détection et le faible coût de l'instrumentation nécessaire pour les utiliser. Ils permettent, au même titre que l'IRM, et dans certaines conditions, de réaliser une détection enzymatique *in vivo.*

D'une manière générale, les substrats enzymatiques fluorescents fonctionnent sur le principe suivant : un substrat non fluorescent dans la gamme de longueur d'onde de détection donne un produit fluorescent dans cette même gamme de longueur d'onde lorsqu'il est mis en présence d'une enzyme dont on souhaite détecter l'activité et qui est spécifique du substrat utilisé. Il est donc nécessaire de trouver des fluorophores dont la fluorescence est inhibée initialement lorsqu'ils sont greffés sur le substrat et qui est capable de se libérer après réaction avec l'enzyme dont on souhaite détecter l'activité. Le choix des fluorophores disponibles commercialement se trouve donc limité par cette contrainte d'inhibition initiale de la fluorescence lorsque le fluorophore est fixé sur le substrat enzymatique.

*In vivo,* le développement récent des méthodes optiques ouvre de nouveaux horizons pour l'imagerie fonctionnelle. Il est maintenant possible de suivre en temps réel et de façon non invasive, l'expression de gènes chez des animaux, en particulier chez la souris, après anesthésie. L'imagerie optique présente un certain nombre d'avantages par rapport aux autres techniques d'imagerie fonctionnelle telles que l'imagerie par résonance magnétique (IRM), l'imagerie par tomographie d'émission de positons (TEP) et l'imagerie par émission monophotonique (SPECT) :
- elle évite la manipulation de molécules radioactives, écartant ainsi les contraintes et les risques qui y sont liés (radioprotection, gestion des déchets, source synchrotron pour les marqueurs TEP) ;
- elle ne nécessite pas de gros investissement d'instrumentation ;
- elle présente une bonne sensibilité par rapport à l'IRM, en terme de quantité de marqueur injectée.

L'imagerie optique met en oeuvre des substrats enzymatiques fluorescents.

Lorsque l'on souhaite détecter la présence d'une activité enzymatique *in vivo,* par exemple chez un petit animal de laboratoire comme la souris, très peu de molécules fluorescentes sont disponibles pour cette application. En effet, pour que la lumière d'excitation et la lumière émise par le fluorophore puissent traverser les tissus, il convient d'utiliser des fluorophores absorbant et émettant dans le proche infrarouge, c'est-à-dire à une longueur d'onde comprise entre 640 et 900 nm. Or, on trouve très peu de molécules fluorescentes dans ce domaine de longueurs d'onde actuellement disponibles commercialement (limitation aux cyanines essentiellement). La double contrainte, à savoir l'inhibition initiale de la fluorescence lorsque le fluorophore est fixé sur le substrat et l'utilisation d'un fluorophore absorbant et émettant dans le proche infrarouge, est sans doute à l'origine de l'absence de substrat enzymatique fluorescent de nature saccharidique dans ce domaine de longueurs d'onde.

En effet, la majorité des substrats enzymatiques fluorescents de nature saccharidique actuellement disponibles sur le marché n'est pas construite à partir de groupements fluorophores absorbant et émettant dans le proche infrarouge. Il est par exemple possible de se procurer :
- des substrats à base de fluorescéine pour la détection de l'activité β-gal parmi lesquels on peut par exemple mentionner le FDG (fluorescein-di-β-D-galactopyranoside) (excitation 490 nm / émission 514 nm) ou l'un de ses dérivés ;
- des substrats à base de coumarines ou d'umbelliferones pour la détection des activités β-gal, β-glu ou phosphatase, tels que les substrats MUG (4-méthylumbelliferone β-D-galactopyranoside), DiFMUG (6,8-difluoro-4-méthylumbelliferyl β-D-galactopyranoside), MUP (4-méthylumbelliferone phosphate), DiFMUP (6,8-difluoro-4-méthylumbelliferyl phosphate) et dérivés (excitation 350-380 nm / émission 450-470 nm) ; ou encore
- des substrats à base de résorufine et dérivés, notamment pour la détection de la lipase (excitation 570 nm / émission 585).

Le document US 2007/09980 décrit de tels substrats enzymatiques fluorescents, ne comprenant pas de groupements fluorophores absorbant et émettant dans le proche infrarouge.

La plupart des substrats fluorescents commercialisés actuellement fonctionnent selon le principe représenté sur le Schéma A ci-après :

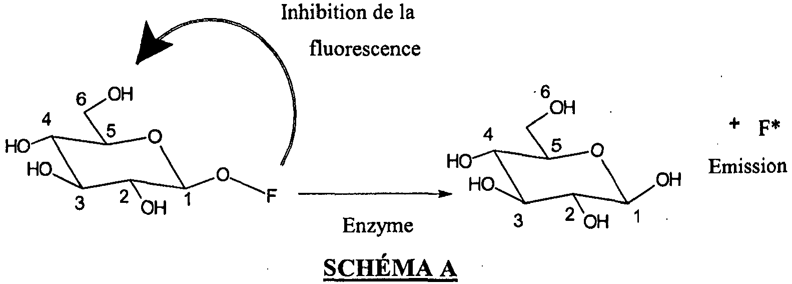

Sur ce schéma, le fluorophore F est greffé en position anomérique 1 (liaison anomérique de configuration β) sur un monosaccharide, le β-glucopyranose, pour former le substrat enzymatique. Ce substrat doit être faiblement fluorescent avant la réaction avec l'enzyme. Les groupements fluorophores doivent donc être choisis de telle sorte que leur fluorescence puisse être inhibée initialement par le monosaccharide. La réaction enzymatique induit un clivage de la liaison anomérique et libère le groupement fluorophore. Lorsque le groupement fluorophore est éloigné du monosaccharide, sa fluorescence n'est plus inhibée et il peut alors émettre un signal qui est détecté à l'aide d'un spectrofluorimètre. Le signal émis reflète l'activité enzymatique et est, dans un certain domaine de concentrations, proportionnel à la concentration en enzyme.

Les substrats fonctionnant selon le principe reporté sur le Schéma A présentent cependant un certain nombre d'inconvénients :
- le choix du groupement fluorophore est limité par le fait que sa fluorescence doit pouvoir être inhibée par son greffage sur le sucre ; tous les groupements fluorophores n'ont pas cette propriété, en particulier dans le domaine du proche infrarouge lorsque l'on souhaite pouvoir faire une détection *in vivo* (voir ci-avant). Ainsi, les substrats fluorogéniques commerciaux comportent toujours des fluorophores des mêmes familles : coumarines, umbelliferones, fluorescéines, résorufines. Notamment, dans le domaine du proche infrarouge, cette limitation est encore plus contraignante, du fait du déjà faible nombre de molécules existantes dans ce domaine de longueurs d'onde.
- si l'inhibition de la fluorescence par le sucre n'est pas complète, la sensibilité de détection du système est médiocre. Pour remédier à ce problème, certains fabricants proposent des substrats dans lesquels le groupement fluorophore est relié à 2 unités saccharidiques par la position anomérique 1. Un exemple de ce type de substrat est la FDG. En multipliant par 2 le nombre d'unités saccharidiques liées au groupement fluorophore, on augmente effectivement l'inhibition initiale de la fluorescence. Néanmoins, la libération du groupement fluorophore et donc de la fluorescence nécessite alors également deux coupures enzymatiques au lieu d'une seule et la sensibilité de la détection n'est donc que peu améliorée dans un tel système.

Le seul substrat enzymatique commercialement disponible absorbant et émettant dans le proche infrarouge est le substrat DDAOG qui est un conjugué de β-galactoside (G) et de 7-hydroxy-9*H*-(1,3-dichloro-9,9-diméthylacridin-2-one) (DDAO) utilisé pour la détection de l'activité β-gal et vendu par la société Molecular Probes (USA). Ce substrat absorbe à 645 nm et émet à 660 nm. Son mode de fonctionnement, tel que décrit par Tung C.-H. et al., Cancer Research, 2004, 64, 1579-1583, est représenté sur le Schéma B ci-après :

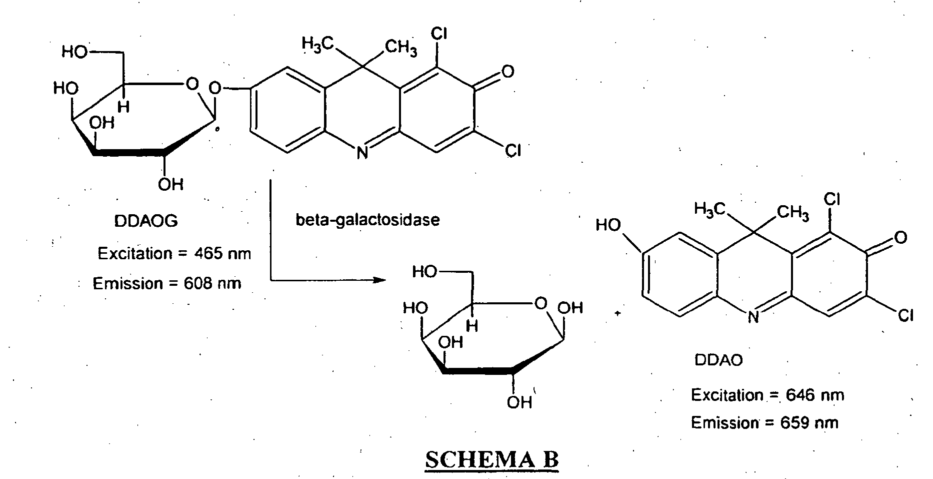

Cependant, bien qu'absorbant et émettant dans le proche infrarouge, ce substrat particulier présente lui aussi un certain nombre d'inconvénients :
- sa fluorescence, lorsque le groupement fluorophore est lié à l'unité saccharidique, n'est pas totalement inhibée, ce qui donne un bruit de fond initialement non négligeable et diminue la sensibilité de détection. Ainsi, on peut constater dans l'expérience *in vivo* relatée dans l'article de Tung C.-H. *et al.,* (précité) que la dose de DDAOG injectée (0,5 mg) et le temps d'exposition nécessaire (2 minutes) sont très importants par rapports aux quantités et temps d'exposition classiques pour ce genre d'applications (en général 10-50 µg de substrat injecté pour un temps d'exposition de 20 à 100 ms) ;
- les spectres d'absorption et d'émission du DDAO sont très étroits et très proches l'un de l'autre, ce qui nécessite un très bon filtrage optique pour la détection du signal par rapport au bruit de fond initial ;
- les spectres d'absorption et d'émission du DDAO ne sont pas encore assez décalés dans le rouge pour être dans une fenêtre optique optimale pour faire de l'imagerie *in vivo.*

Il a également déjà été proposé, notamment dans la demande de brevet FR 2 888 938, des substrats fluorescents comportant un squelette saccharidique portant, sur la même unité saccharidique, un groupement fluorophore d'une part, et un groupement inhibiteur de la fluorescence du groupement fluorophore d'autre part, étant entendu que l'un de ces deux groupements occupe la position anomérique de l'unité saccharidique sur laquelle ils sont tous les deux fixés, l'autre groupement occupant n'importe quelle autre position de l'unité saccharidique.

De tels substrats fluorescents permettent la fixation d'une plus grande variété de groupements fluorophores absorbant et émettant dans le proche infrarouge, et donc utilisables *in vivo.* Cependant, ils ne donnent pas non plus entièrement satisfaction, en particulier parce que l'affinité des enzymes pour de tels substrats s'en trouve très amoindrie et rend le processus trop peu efficace.

Enfin, et très récemment, il a été développé une sonde saccharidique appelée Gal-2SBPO, résultant de la conjugaison du substrat de la β-galactosidase (le β-D-galactopyranoside : Gal) et d'un colorant hydrosoluble fluorescent, le perchlorate de 9-di-3-disulfonyl propylaminobenzo[a]phénoxazonium (2SBPO) par l'intermédiaire d'un bras espaceur incluant un peptide. Dans ce substrat enzymatique (Gal-2SBPO), la fluorescence est inhibée par le peptide (glycine) inclus dans le bras espaceur reliant l'unité saccharidique Gal et le groupement fluorophore 2SBPO proprement dit (HO, N.-H. et al., Chem. Bio. Chem., 2007, 8, 560-566). L'activité enzymatique de la β-galactosidase induit un clivage entre le sucre et le bras espaceur, le groupement fluorophore pouvant alors fluorescer après une ultime hydrolyse qui le sépare de son peptide inhibiteur. Le mode de fonctionnement de cette sonde enzymatique peut être représenté par le schéma C suivant :

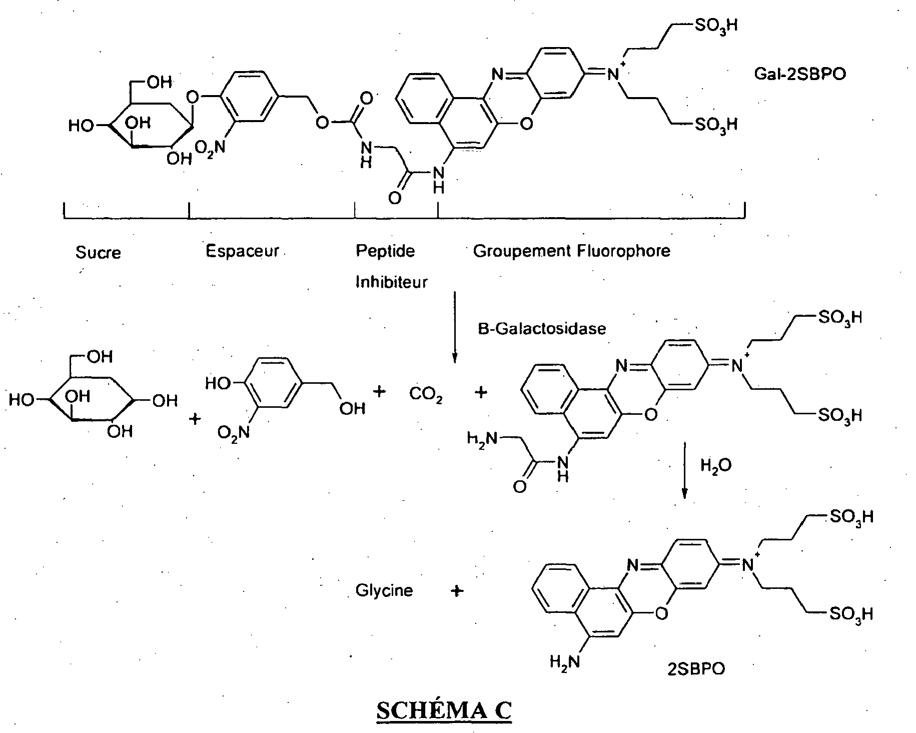

Cependant, un tel système présente encore un certain nombre de limitations :
- le choix du fluorophore se restreint uniquement au perchlorate de 9-di-3-disulfonyl propylaminobenzo[a]phénoxazonium qui est aujourd'hui, semble t-il, le seul groupement fluorophore dont la fluorescence peut être inhibée par la présence d'un acide aminé tel que la glycine (HO, N.-H. et al., Tetrahedron, 2006, 62, 578-585).
- l'intensité de la fluorescence, après l'activité enzymatique, n'est accrue que d'un facteur 7, ce qui représente encore un faible rapport pour envisager d'utiliser ce marqueur fluorescent en imagerie *in vivo* en l'état actuel des possibilités des instruments de mesure.

C'est donc afin de remédier à l'ensemble de ces problèmes que les Inventeurs ont mis au point ce qui fait l'objet de l'invention.

Les Inventeurs se sont en effet fixés pour but de pourvoir à un substrat enzymatique fluorescent de nature saccharidique n'ayant pas les inconvénients des substrats de l'art antérieur et qui soit en particulier bien adapté, lorsqu'on le souhaite, à une utilisation pour la détection d'activités enzymatiques *in vivo.*

La présente invention a donc pour objet un substrat enzymatique fluorescent tel que défini dans les revendications annexées.

Plus précisément, le substrat enzymatique de l'invention est caractérisé par le fait qu'il répond à la structure (I) suivante :

S-B(I)ₙ-F (I)

dans laquelle :
- S est un squelette de nature saccharidique constitué d'au moins une unité saccharidique et choisi parmi les monosaccharides, les oligosaccharides ayant de 2 à 9 unités saccharidiques et les polysaccharides ayant au moins 10 unités saccharidiques ;
- F est un groupement fluorophore porté par le bras espaceur B ;
- I est un groupement inhibiteur de la fluorescence de F ; ledit groupement I étant un substituant latéral d'au moins une sous-unité du bras espaceur B ;
   étant entendu qu'aucune liaison covalente ne relie directement le groupement fluorophore au groupement inhibiteur.
- n est un nombre entier égal à 1 ou 2 ;
- B représente un bras espaceur autosécable constitué d'une ou de plusieurs sous-unités ; ledit bras B étant fixé en position anomérique de ladite unité saccharidique S, la liaison anomérique étant indifféremment en position α ou β, ledit bras B correspondant à un site reconnu et clivé par une enzyme et comprenant une sous-unité B1 choisie parmi :
   i) les groupements aromatiques monocycliques de formule (II) suivante : 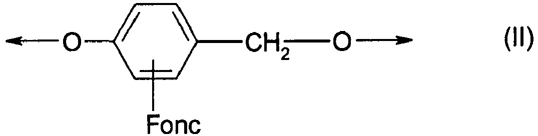 dans laquelle :
      - Fonc est une fonction chimique réactive vis-à-vis d'une fonction chimique complémentaire d'un groupement fluorophore F ou respectivement d'un groupement inhibiteur de la fluorescence d'un groupement fluorophore F,
      - la flèche partant de l'atome d'oxygène directement porté par le cycle phényle représente le point d'attachement de ladite sous-unité B1 avec une unité saccharidique du bras espaceur par l'intermédiaire d'une liaison covalente avec l'atome de carbone situé en position 1 anomérique de ladite unité saccharidique,
      - la flèche partant de l'atome d'oxygène lié au radical -CH₂-représente le point d'attachement de ladite sous-unité B1 avec une sous-unité B2 choisie parmi les groupements aromatiques de formule (III) suivante : 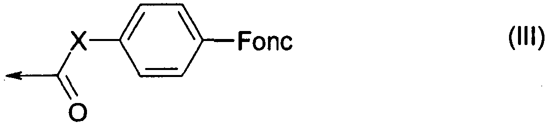 dans laquelle :
         - Fonc est une fonction chimique réactive vis-à-vis d'une fonction chimique complémentaire d'un groupement inhibiteur de la fluorescence du groupement F ou respectivement d'un groupement fluorophore F ;
         - la flèche représente le point d'attachement de ladite sous-unité B2 avec la sous-unité B1 par l'intermédiaire d'une liaison covalente avec l'atome d'oxygène de la sous-unité B1,
         - X est O, NH ou S; et
   ii) les groupements aromatiques monocycliques de formule (IV) suivante : 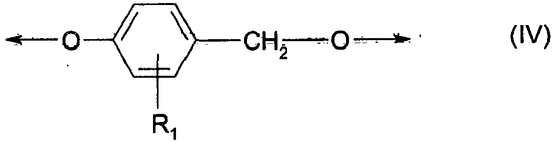 dans laquelle :
      - R₁ est choisi parmi les groupements nitro, sulfate, amine et amine protégée par un groupement protecteur,
      - la flèche partant de l'atome d'oxygène directement porté par l'atome de carbone du cycle phényle représente le point d'attachement de ladite sous-unité B1 avec une unité saccharidique du bras espaceur par l'intermédiaire d'une liaison covalente avec l'atome de carbone situé en position 1 anomérique de ladite unité saccharidique,
      - la flèche partant de l'atome d'oxygène lié au radical -CH₂-représente le point d'attachement de ladite sous-unité B1 avec une sous-unité B2 choisie parmi les groupements de formule (V) suivante : 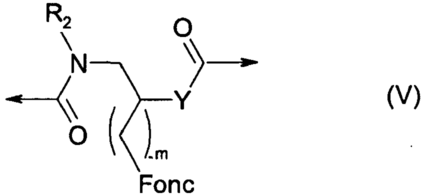 dans laquelle :
         - R₂ est un atome d'hydrogène ou un radical alkyle en C₁-C₄,
         - Fonc est une fonction chimique réactive vis-à-vis d'une fonction chimique complémentaire d'un groupement fluorophore F ou respectivement d'un groupement inhibiteur de la fluorescence d'un groupement fluorophore F ;
         - m est un nombre entier allant de 1 à 10 ;
         - Y est O, NH ou S ;
         - les flèches représentent le point d'attachement de ladite sous-unité B2 avec d'une part la sous-unité B1 et d'autre part avec un atome d'azote ou d'oxygène porté par une sous-unité B3 de formule (VI) suivante : 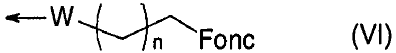 dans laquelle :
            - W représente O, NH ou S ;
            - la flèche représente le point d'attachement de l'atome d'azote, de soufre ou d'oxygène désigné pour W, par l'intermédiaire d'une liaison covalente avec un atome de carbone de la sous-unité B2,
            - n est un nombre entier allant de 1 à 10 ;
            - Fonc est une fonction chimique réactive vis-à-vis d'une fonction chimique complémentaire d'un groupement fluorophore F ou respectivement d'un groupement inhibiteur de la fluorescence du groupement fluorophore F.Dans ces substrats enzymatiques de formule (I) ci-dessus, le bras espaceur B autosécable reliant le squelette saccharidique au groupement fluorophore F présente la particularité de se disloquer spontanément en une ou plusieurs sous-unités distinctes après l'activité enzymatique, c'est-à-dire après que l'enzyme ait entraîné la coupure de la liaison reliant ledit bras espaceur au squelette saccharidique. Cette dislocation provoque la rupture de la liaison reliant, d'une part, le bras espaceur au groupement fluorophore, et d'autre part de la liaison reliant le bras espaceur au groupement inhibiteur de la fluorescence, libérant ainsi la fluorescence du groupement F.

Les substrats enzymatiques conformes à l'Invention et tels que décrits ci-dessus présentent une très bonne inhibition initiale de la fluorescence compte tenu de la présence d'un ou de deux groupements I à titre de substituant latéral du bras espaceur. Cette configuration particulière permet, par rapport aux substrats connus dans l'état de la technique :
- d'élargir le choix des groupements fluorophores utilisables : le choix d'un tel groupement n'est plus limité aux groupements dont la fluorescence doit initialement être inhibée par le squelette S ou par un peptide faisant partie intégrante d'un bras espaceur. Le choix des groupements fluorophores disponibles est donc beaucoup plus large et peut notamment s'étendre aux groupements fluorophores émettant dans le proche infrarouge ;
- d'accroître encore la sensibilité de détection de l'activité enzymatique : en effet, la fluorescence du groupement fluorophore est inhibée par un objet dont c'est spécifiquement le rôle et qui est ainsi beaucoup plus efficace que lorsque l'inhibition doit être réalisée par le squelette saccharidique ou un peptide ;
- de permettre une meilleure affinité pour l'enzyme cible : en effet, le fait de greffer le fluorophore et l'inhibiteur de fluorescence sur la partie aglycone sécable permet une meilleure affinité pour l'enzyme puisque tous les sites de reconnaissance sont libres,
- d'accroître la solubilité du substrat par le biais de l'utilisation de groupements fluorophores beaucoup plus solubles que les groupements fluorophores actuellement disponibles dans le commerce et qui sont généralement hydrophobes,
- de faciliter les conditions d'utilisation du substrat enzymatique, par le biais de l'utilisation de fluorophores par exemple insensibles au pH ou au potentiel redox, et donc plus facilement utilisables *in vivo.* En effet, certains fluorophores utilisés actuellement, telle la fluorescéine, ont des propriétés d'émission très dépendantes du pH, et difficilement compatibles avec une utilisation *in vivo* où les conditions intra-cellulaires ne peuvent être modifiées. En utilisant d'autres fluorophores dont les propriétés d'émission sont peu dépendantes du pH, du potentiel redox ou de la concentration en ions, les conditions d'acquisition du signal et la sensibilité *in vivo* sont facilitées.

Les unités saccharidiques du squelette S des substrats de structure (I) conformes à l'Invention, peuvent notamment être choisies parmi le galactose, le mannose, l'idose, le talose, le rhamnose, le glucose, le ribose, le fucose et leurs dérivés aminés ou acides parmi lesquels on peut notamment citer la galactosamine, la glucosamine, la lactosamine, l'acide glucuronique, l'acide iduronique et l'acide sialique. Elles sont de préférence choisies parmi la glucosamine, le galactose et l'acide glucuronique.

Lorsque le squelette S est un monosaccharide, ces unités saccharidiques sont utilisées unitairement. Elles sont par contre reliées entre elles par des liaisons glycosidiques lorsque le squelette S est un oligosaccharide ou un polysaccharide.

Selon l'Invention, lorsque le squelette S est un oligosaccharide, il est de préférence choisi parmi les oligosaccharides comportant de 4 à 9 unités saccharidiques.

Les positions libres des unités saccharidiques du squelette S, qui ne comportent pas le bras espaceur, et qui ne sont pas engagées dans une liaison glycosidique, peuvent indifféremment être non substituées (-H ou -OH) ou bien être substituées par exemple par une fonction amine ou par un groupement résultant de l'interaction d'une fonction hydroxyle ou d'une fonction amine avec un groupement protecteur tel que ceux classiquement utilisés en chimie organique et décrits par exemple, dans l'ouvrage de T. W. Greene et al., "Protective Groups in Organic Synthesis", Third Edition, Wiley Science (1999). Parmi de tels groupements protecteurs, on peut notamment citer les groupements acétyle ; benzyle ; aryle et en particulier les groupements aryle substitués par un radical choisi parmi les chaînes alkyle ayant de 1 à 40 atomes de carbone ; 2,2,2-trichloroéthyloxycarbonyle (Troc) ; benzyloxycarbonyle (BzC) ; trichloroacétamidate (TCA) ; tert-butyloxycarbonyle (BOC), fluoranylméthoxycarbonyle (Fmoc), ainsi que les groupements silylés tels que par exemple les groupements t-butyldiméthylsilyle (tBDMS) et triméthylsilyle (TMS), ou bien encore des chaînes de polyéthylène glycol (PEG). De façon avantageuse, la présence d'un groupement acétyle peut permettre une meilleure pénétration des substrats dans les cellules et la présence d'un PEG permet par ailleurs d'ajuster les propriétés pharmacocinétiques des substrats conformes à l'Invention.

La ou les sous-unités pouvant constituer le bras espaceur des substrats enzymatiques de formule (I) est une chaîne hydrocarbonée linéaire ou ramifiée, saturée ou insaturée, substituée ou non substituée, interrompue et/ou terminée par un ou plusieurs hétéroatomes choisis parmi N, O et S, et/ou par un ou plusieurs groupements choisis parmi les radicaux alkyle en C₁-C₄, alcoxy en C₁-C₄, aryle ou par une ou plusieurs fonctions choisies parmi les fonctions éther, ester, amide, carbonyle, carbamate, urée, thiourée et disulfure.

Sont de fait exclus de cette définition, tous les bras espaceurs qui comporteraient (ou seraient constitués d') une chaîne phosphate, une unité saccharidique ou une base azotée.

Le bras espaceur peut encore être constitué constitué d'au moins deux sous-unités bifonctionnelles B1 et B2 telles que l'une des extrémités de la sous-unité B1, respectivement B2, est une fonction réactive vis-à-vis des groupements classiques d'activation de la position anomérique de l'unité saccharidique sur laquelle elles doivent être fixées (exemples notables de groupes d'activation de la position anomérique : -Br, -SPh avec Ph = phényle ; exemple notable de fonction réactives vis-à-vis de ces groupements d'activation : -OH en particulier), l'autre extrémité de la sous-unité B1 étant une fonction (par exemple amine ou thiol) réactive vis-à-vis d'une fonction complémentaire portée par l'une des extrémités de la sous-unité B2, laquelle sous-unité B2 comportant, à son autre extrémité, une fonction (par exemple amine ou thiol) réactive vis-à-vis d'une fonction de greffage portée par le groupement fluorophore F (telle que par exemple une fonction N-hydroxysuccinimidyle, isothiocyanate, ester de sulfotétrafluorophényl (STP-ester), maléimide ou haloacétamide).

Mais, selon une première forme de réalisation de l'invention, le bras espaceur comprend une sous-unité B1 choisie parmi les groupements aromatiques monocycliques de formule (II) suivante : 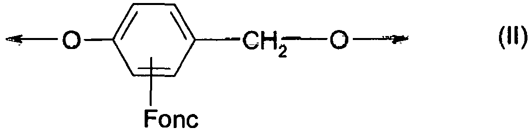 dans laquelle :
- Fonc est une fonction chimique réactive vis-à-vis d'une fonction chimique complémentaire d'un groupement fluorophore F ou respectivement d'un groupement inhibiteur de la fluorescence d'un groupement fluorophore F,
- la flèche partant de l'atome d'oxygène directement porté par le cycle phényle représente le point d'attachement de ladite sous-unité B1 avec une unité saccharidique du bras espaceur par l'intermédiaire d'une liaison covalente avec l'atome de carbone situé en position 1 anomérique de ladite unité saccharidique,
- la flèche partant de l'atome d'oxygène lié au radical -CH₂-représente le point d'attachement de ladite sous-unité B1 avec un groupement inhibiteur de la fluorescence du groupement F ou avec une sous-unité B2 choisie parmi les groupements aromatiques de formule (III) suivante : 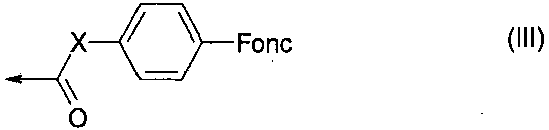 dans laquelle :
   - Fonc est une fonction chimique réactive vis-à-vis d'une fonction chimique complémentaire d'un groupement inhibiteur de la fluorescence du groupement F ou respectivement d'un groupement fluorophore F ;
   - la flèche représente le point d'attachement de ladite sous-unité B2 avec la sous-unité B1 par l'intermédiaire d'une liaison covalente avec l'atome d'oxygène de la sous-unité B1,
   - X est O, NH ou S.

Dans la sous-unité B 1 de formule (II) ci-dessus, le groupement Fonc est de préférence en position ortho par rapport à l'atome de carbone cyclique porteur de l'atome d'oxygène.

Selon une seconde forme de réalisation de l'Invention, le bras espaceur comprend une sous-unité B1 choisie parmi les groupements aromatiques monocycliques de formule (IV) suivante : 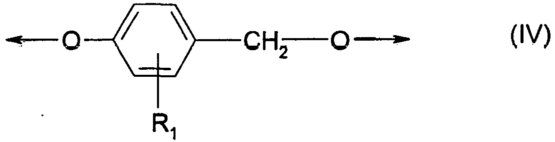 dans laquelle :
- R₁ est choisi parmi les groupements nitro, sulfate, amine et amine protégée par un groupement protecteur,
- la flèche partant de l'atome d'oxygène directement porté par l'atome de carbone du cycle phényle représente le point d'attachement de ladite sous-unité B1 avec une unité saccharidique du bras espaceur par l'intermédiaire d'une liaison covalente avec l'atome de carbone situé en position 1 anomérique de ladite unité saccharidique,
- la flèche partant de l'atome d'oxygène lié au radical -CH₂- représente le point d'attachement de ladite sous-unité B1 avec une sous-unité B2 choisie parmi les groupements de formule (V) suivante : 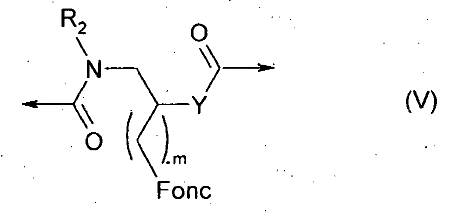
dans laquelle :
- R₂ est un atome d'hydrogène ou un radical alkyle en C₁-C₄,
- Fonc est une fonction chimique réactive vis-à-vis d'une fonction chimique complémentaire d'un groupement fluorophore F ou respectivement d'un groupement inhibiteur de la fluorescence d'un groupement fluorophore F ;
- m est un nombre entier allant de 1 à 10 ;
- Y est O, NH ou S ;
- les flèches représentent le point d'attachement de ladite sous-unité B2 avec d'une part la sous-unité B1 et d'autre part avec un atome d'azote ou d'oxygène porté par une sous-unité B3 de formule (VI) suivante : 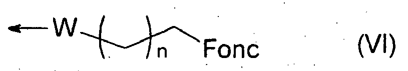
dans laquelle :
- W représente O, NH ou S;
- la flèche représente le point d'attachement de l'atome d'azote, de soufre ou d'oxygène désigné pour W, par l'intermédiaire d'une liaison covalente avec un atome de carbone de la sous-unité B2,
- n est un nombre entier allant de 1 à 10 ;
- Fonc est une fonction chimique réactive vis-à-vis d'une fonction chimique complémentaire d'un groupement fluorophore F ou respectivement d'un groupement inhibiteur de la fluorescence du groupement fluorophore F.
Ainsi, en fonction de la nature des atomes désignés pour X, Y, et Z les différentes sous-unité de formules (II) à (VI) pouvant constituer le bras espaceur des substrats enzymatiques de formule (I) conformes à la présente Invention, le bras espaceur comprend une ou plusieurs fonctions amide, carbonate, carbamate, urée ou thiourée qui sont aptes à s'hydrolyser spontanément après clivage enzymatique de la liaison avec l'unité saccharidique. De façon plus précise :
- la fonction reliant, entre elles, les sous-unités B1 de formule (II) et B2 de formule (III) peut être une fonction carbonate (X = O) ou une fonction carbamate (X = NH) ;
- la fonction reliant, entre elles, les sous-unités B1 de formule (IV) et B2 de formule (V) est une fonction carbamate ;
- la fonction reliant, entre elles, les sous-unités B2 de formule (V) et B3 de formule (VI) peut être une fonction carbonate (Y = W = O), carbamate (Y = O avec W = NH ou Y = NH avec W = O), urée (Y = W = NH), ou encore thiourée (Y=S avec W=NH ou Y=NH avec W=S).

Dans les sous-unités de formules (II), (III), (V) et (VI), la fonction Fonc est de préférence choisie parmi les fonctions amine primaire et thiol.

Parmi ces fonctions, la fonction amine primaire est particulièrement préférée.

Parmi les sous-unités de formule (II) ci-dessus, on préfère celles dans lesquelles Fonc est située en position ortho de l'atome de carbone porteur de l'atome d'oxygène.

Parmi les sous-unités de formule (III) ci-dessus, on préfère celles dans lesquelles :
- X est un atome d'oxygène et Fonc est une fonction amine primaire ou thiol,
- X est un atome d'azote et Fonc est une fonction amine primaire ou thiol.

Parmi les sous-unités de formule (IV) ci-dessus, on préfère celles dans lesquelles R₁ est situé en position ortho de l'atome de carbone porteur de l'atome d'oxygène.

Parmi les sous-unités de formule (V) ci-dessus, on préfère celles dans lesquelles :
- R₂ est un radical méthyle, m = 1, Fonc est une fonction amine primaire et Y est un atome d'oxygène,
- R₂ est un radical méthyle, m = 1, Fonc est une fonction amine primaire et Y = NH.

Parmi les sous-unités de formule (VI) ci-dessus, on préfère celles dans lesquelles :
- W est un atome d'oxygène, n = 1, Fonc est une fonction amine primaire et Y est un atome d'oxygène,
- W représente NH, n = 1, Fonc est une fonction amine primaire et Y est un atome d'oxygène,
- W est un atome de soufre, n = 1, Fonc est une fonction amine primaire et Y est un atome d'oxygène.

Selon une forme de réalisation particulièrement préférée de l'Invention, les substrats enzymatiques de formule (I) conformes à l'Invention sont choisis parmi les composés de formules (I-1) et (I-2) suivantes : 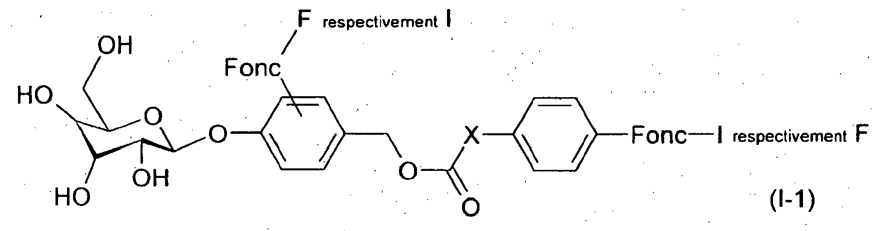 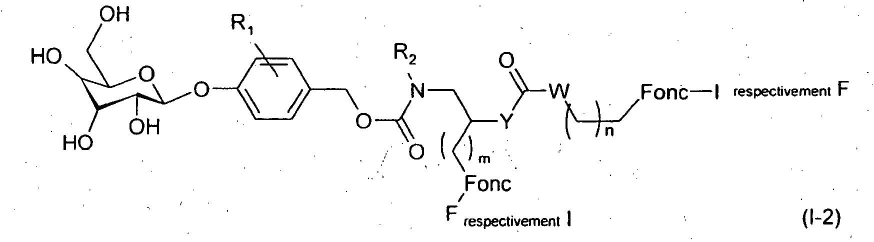 dans lesquelles, F, I, Fonc, X, Y, W, R₁ R₂, m et n ont les mêmes significations que celles indiquées précédemment.

Parmi les groupements fluorophores F, on peut notamment citer la fluorescéine (fluorescéinate de sodium) et ses dérivés tels que l'isothiocyanate de fluorescéine (FITC) et la 6-carboxyfluorescéine (6-Fam) ; les colorants fluorescents absorbant et émettant dans le proche infrarouge ("Near InfraRed" : NIR) tels que ceux vendus sous les dénominations Fluorescent Red NIR 700 (longueur d'onde d'excitation : 672 nm ; longueur d'émission : 735 nm) et Fluorescent Red NIR 730 (longueur d'onde d'excitation : 680 nm ; longueur d'émission : 755 nm) par la société Sigma-Aldrich ; les cyanines fluorescentes telles que le Cy5 (n=2) et le Cy7 (n = 3) (Amersham) ; le 7-hydroxy-9*H-*(1,3-dichloro-9,9-diméthylacridin-2-one) (DDAO) ; la rhodamine et ses dérivés tels que la tetraméthyl rhodamine isothiocyanate (TRITC) ; les colorants fluorescents à amines réactives telles que les coumarines parmi lesquelles on peut notamment citer l'ester succinimidyl de l'acide 6-((7-amino-4-méthylcoumarin-3-acétyl)amino) hexanoïque (AMCA) ; les difluorures de bore de dipyrrométhène vendus sous les dénominations commerciales BODIPY ® tels que BODIPY® FR-Br₂, BODIPY ® R6G, BODIPY® TMR, BODIPY ® TR et les BODIPY ® 530/550 (longueur d'onde d'excitation/longueur d'onde d'émission, en nm), 558/568, 564/570, 576/589, 581/591, 630/650 et 650/665 vendus par la société Bio-Rad Inc. (USA), IRDye ® 800 vendu par la société LICOR et Alexa Fluor ® 750 vendu par la société Molecular Probes ; les porphyrines ; les cyanines ; les oxazines ; les fluorophores dérivés du pyrène tels que par exemple les colorants Cascade Blue (vendus par exemple par les sociétés Trilink BioTechnologies (USA) ou Invitrogen ; les dérivés diazoïques tels que le DABCYL® ; les dérivés du dansyle tels que l'EDANS® (Eurogentec, BE) ; l'éosine ; l'érythrosine et les dérivés de sulforhodamine tels que le chlorure/sulfonyl de sulforhodamine 101 également connu sous le nom de Texas Red ; et les nanoparticules fluorescentes c'est-à-dire ayant des propriétés d'émission telles que les "quantum dots", les nanoparticules d'or, les nanoparticules à base de polymères et les nanoparticules d'oxydes.

Selon une forme de réalisation particulièrement préférée de l'Invention, le groupement F est choisi parmi les groupements fluorophores absorbant et émettant dans le proche infrarouge, c'est-à-dire émettant et absorbant à une longueur d'onde comprise entre 640 et 900 nm. Parmi de tels groupements, on peut en particulier citer les groupements fluorophores suivants : les colorants fluorescents vendus sous les dénominations Fluorescent Red NIR 700 (longueur d'onde d'excitation (Ex.) : 672 nm / longueur d'émission (Em.) : 735 nm) et Fluorescent Red NIR 730 (Ex : 680 nm / Em : 755 nm) par la société Sigma-Aldrich ; le Cy5 (n = 2 ; Ex. : 680 nm / Em. : 755 nm), le Cy5.5 (Ex. : 675 nm / Em. : 694 nm) et le Cy7 (n = 3 : Ex. : 747 nm / Em. : 775 nm) (Amersham) ; le 7-hydroxy-9*H*-(1,3-dichloro-9,9-diméthylacridin-2-one) (DDAO) (Ex. : 646 nm / Em. : 659 nm), l'IRDye ® 800 (Ex. : 778 / EM. : 806 nm), l'Alexa Fluor ® 647 (Ex. : 651 nm / Em. : 672 nm), l'Alexa Fluor ® 660 (Ex. : 668 nm / Em. : 698 nm), l'Alexa Fluor ® 680 (Ex. : 684 nm / Em. : 707 nm), et l'Alexa Fluor ® 700 (Ex. : 702 nm / Em. : 723 nm) et l'Alexa Fluor ® 750 (Ex. : 749 nm / Em. : 774 nm).

Selon une forme de réalisation particulière de l'invention, le groupement fluorophore F peut en outre être fonctionnalisé par un ou plusieurs fonctions amide ou urée et/ou par un ou plusieurs groupements choisis parmi les chaînes lipophiles, les phospholipides et les peptides. Ainsi fonctionnalisés, les groupements fluorophores F présentent une plus grande affinité pour les cellules des tissus animaux.

Selon l'Invention, le groupement I peut être choisi parmi tous les composés acceptant la fluorescence du groupement F, c'est à dire permettant la diminution ou la disparition complète de la fluorescence du groupement F lorsqu'ils sont tous deux fixés sur le bras espaceur des substrats enzymatiques de formule (I). Ce composé, de natures diverses, peut notamment être un groupement chromophore, fluorescent ou non fluorescent, ou une nanoparticule.

Lorsque le groupement I est lui-même un groupement fluorescent, alors il est choisi parmi les groupements dont la fluorescence inhibe celle du groupement F. Le groupement I peut être identique au groupement F (on parle alors d'auto-inhibition de la fluorescence) et est de préférence choisi parmi les cyanines fluorescente telles que Cy5, Cy5.5 et Cy7, l'IRDye ® 800 et les Alexa Fluor ® 647, 660, 680, 700 et 750. A titre de groupement I, on peut également utiliser un groupement fluorescent, différent du groupement F, qui absorbe la fluorescence du groupement F par transfert d'énergie par résonance de fluorescence (FRET). Dans ce cas on préfère utiliser les couples F/I suivants : Cy5/Cy7 (ou Cy7Q); Cy5/Alexa Fluor ® 750 ; Cy7/IRDye ® 800 ; Alexa Fluor ® 750/IRDye ® 800.

Lorsque le groupement I est un groupement non-fluorescent, c'est à dire un inhibiteur de fluorescence proprement dit ("Quencher"), alors il est de préférence choisi parmi les composés vendus sous les dénominations commerciales DABCYL ® et dérivés, Black Hole Quencher ® (BHQ) telles que BHQ 1, BHQ 2 ou BHQ 3 (Biosearch Technologies), Nanogold Particules ® (Nanoprobes), Eclipse Dark Quencher ® (Epoch Bioscience), Elle Quencher ® (Oswell), Cy7Q (Amersham) et les colorants QSY ® tels que les QSY ® 7, QSY ® 9 et QSY ® 21 (Molecular Probes).

Parmi les substrats enzymatiques de formule (I) conformes à l'Invention, on préfère tout particulièrement les composés de formule (I) dans lesquels :
i) le squelette S est une galactosamine, le bras espaceur est constitué d'une sous-unité B1 de formule (II) et d'une sous-unité B2 de formule (III) et F et I sont identiques. Dans ce cas F et I sont choisis par exemple parmi les groupements Cy5, Cy5.5 et Cy7, les Alexa Fluor ® 647, 660, 680, 700 et 750, et IRDye ® 800, ces groupements étant respectivement et indifféremment portés par les sous-unités B1 et B2;
ii) le squelette S est une galactosamine, le bras espaceur est constitué d'une sous-unité B1 de formule (IV), d'une sous-unité B2 de formule (V) et d'une sous-unité B3 de formule (VI) et F et I sont identiques. Dans ce cas F et I sont choisis par exemple parmi les groupements Cy5, Cy5.5 et Cy7, les Alexa Fluor ® 647, 660, 680, 700 et 750, et IRDye ® 800, ces groupements étant respectivement et indifféremment portés par les sous-unités B2 et B3 ;
iii) le squelette S est une galactosamine, le bras espaceur est constitué d'une sous-unité B1 de formule (IV), et d'une sous-unité B2 de formule (III) et F et I sont différents. Dans ce cas F et I sont choisis par exemple parmi les couples de groupements suivants Cy5/Cy7 (ou Cy7Q) ; Cy5/Alexa Fluor ® 750 ; Cy7/IRDye ® 800 ; Alexa Fluor ® 750/IRDye ® 800, ces groupements étant respectivement et indifféremment portés par les sous-unités B2 et B3.

Selon une forme de réalisation particulièrement préférée de l'Invention, les substrats enzymatiques de formule (I-1) sont choisis parmi les composés suivants :

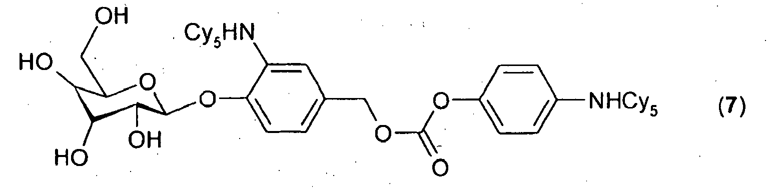

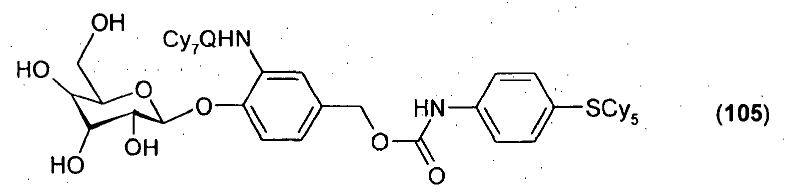

A titre de substrats enzymatiques de formule (I-2), on peut en particulier mentionner le composé suivant :

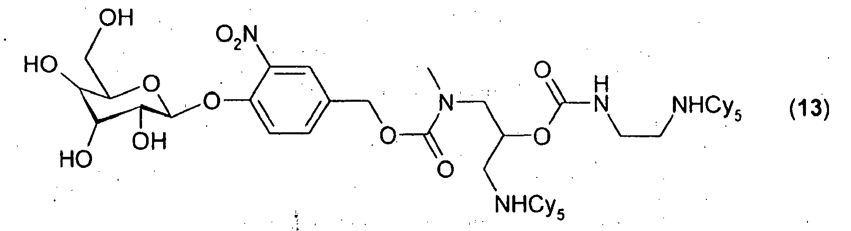

Les substrats enzymatiques de formule (I) conformes à l'Invention peuvent être préparés facilement suivant un enchaînement d'étapes distinctes classiques et connues de l'homme du métier.

En particulier, les substrats enzymatiques de formule (I) conformes à l'invention dans lesquels les sous-unités B1 et B2 sont choisies respectivement parmi les sous-unités de formules (II) et (III) (composés de formule (I-1)) et dans lesquels F et I représentent un groupement fluorophore identique, peuvent par exemple être préparés selon le procédé (P1) tel que représenté sur le schéma D suivant : 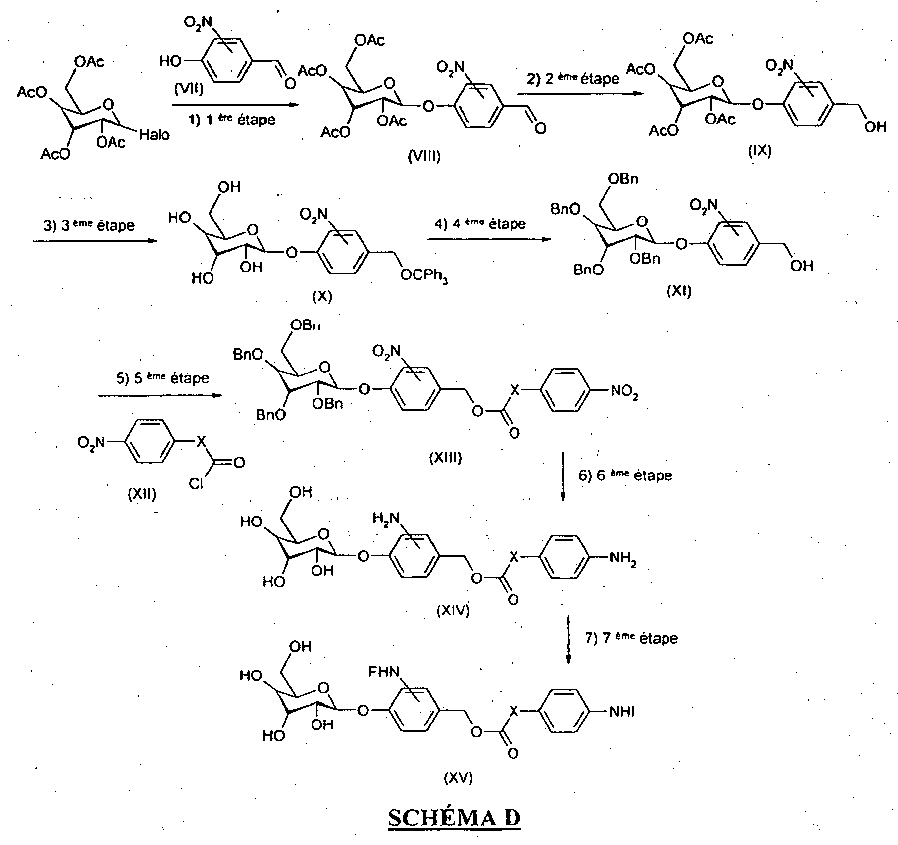 selon lequel :
1) dans une première étape, on fait réagir, dans un solvant organique tel que par exemple de l'acétonitrile anhydre, et en présence d'un agent oxydant, un squelette saccharidique comportant au moins une unité saccharidique dans laquelle la position 1 anomérique est halogénée et dont les fonctions hydroxyle sont acétylées, avec un 4-hydroxynitrobenzaldéhyde de formule (VII) pour conduire à un composé de formule (VIII) dans lequel "Ac" signifie acétyle, étant entendu que dans les composés de formules (VII) et (VIII) le groupement nitro peut occuper n'importe quelle position du cycle benzénique ;
2) dans une deuxième étape, on effectue, dans un solvant organique tel que par exemple le chloroforme, l'isopropanol ou leur mélange, une réduction du composé de formule (VIII) obtenu ci-dessus à l'étape précédente, en présence d'un agent réducteur pour conduire à un composé de formule (IX) ;
3) dans une troisième étape, on fait réagir, dans un solvant organique tel que par exemple du dichlorométhane, le composé de formule (IX), obtenu ci-dessus à l'étape précédente, avec du chlorure de trityle, puis on hydrolyse les groupements acétyle pour conduire à un composé de formule (X) dans lequel "Ph" signifie phényle ;
4) dans une quatrième étape, on fait réagir, dans un solvant organique tel que par exemple diméthylformamide, le composé de formule (X) obtenu ci-dessus à l'étape précédente, avec un halogénure de benzyle puis on hydrolyse le groupement trityle pour libérer la fonction hydroxyle et conduire à un composé de formule (XI) dans lequel "Bn" signifie benzyle ;
5) dans une cinquième étape, on fait réagir, dans un solvant organique tel que le dichlorométhane, le composé de formule (XI) obtenu ci-dessus à l'étape précédente avec un composé de formule (XII) pour conduire à un composé de formule (XIII) dans lequel "Bn" signifie benzyle ; étant entendu que dans les composés de formule (XII) et (XIII), X représente O ou NH ;
6) dans une sixième étape, on effectue une réduction des groupements nitro du composé de formule (XIII) obtenu ci-dessus à l'étape précédence en présence d'un agent réducteur, pour conduire à un composé de formule (XIV) ; et
7) dans une septième étape, on fait réagir, dans un solvant organique tel que par exemple du diméthylformamide anhydre, le composé de formule (XIV) obtenu ci-dessus à l'étape précédente, avec un composé fluorophore préalablement fonctionnalisé par un groupement NHS, pour conduire à un composé de formule (XV) dans lequel F = I (composé de type (I-1)).

D'autre part, les substrats enzymatiques de formule (I) conformes à l'invention dans lesquels le bras espaceur comprend au moins une sous-unité B1 de formule (IV), une sous-unité B2 de formule (V) et une sous-unité B3 de formule (VI) (composés de formule (I-2)) et dans lesquels F et I représentent un groupement fluorophore identique, peuvent par exemple être préparés selon le procédé (P2) tel que représenté sur le schéma E suivant : 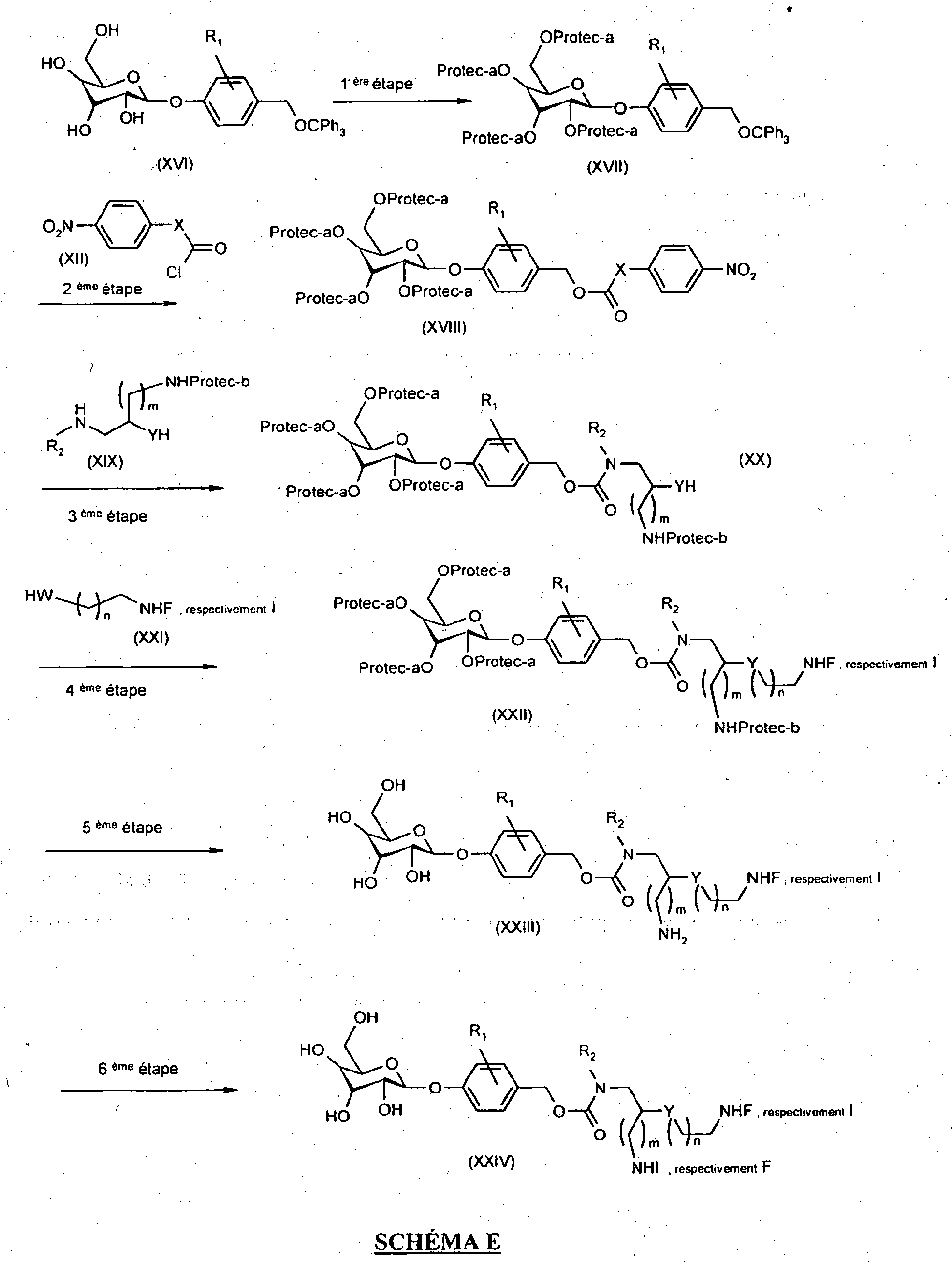 selon lequel :
1) dans une première étape, on fait réagir, dans un solvant organique tel que par exemple du dichlorométhane, un composé de formule (XVI) dans lequel R₁ a la même signification que celle indiquée précédemment pour la sous-unité B1 de formule (IV) et "Ph" signifie phényle, avec un groupement protecteur "Protec-a" tel que par exemple le *tert*-butyldiméthylchlorosilane (TBDMS) en présence d'un composé imidazole, pour conduire à un composé de formule (XVII) ;
2) dans une deuxième étape, on fait réagir, dans un solvant organique tel que par exemple le dichlorométhane, le composé de formule (XVII) obtenu ci-dessus à l'étape précédente, avec un composé de formule (XII) tel que défini ci-dessus à la cinquième étape du procédé P1, pour conduire à un composé de formule (XVIII) ;
3) dans une troisième étape, on fait réagir, dans un solvant organique tel que par exemple le dichlorométhane, le composé de formule (XVIII) obtenu ci-dessus à l'étape précédente, avec un composé de formule (XIX) dans lequel R₂, Y et m ont les mêmes significations que celles indiquées précédemment pour la sous-unité B2 de formule (V) et Protec-b est un groupement protecteur tel que par exemple le Fmoc, pour conduire à un composé de formule (XX) ;
4) dans une quatrième étape, on fait réagir, dans un solvant organique tel que par exemple le dichlorométhane anhydre, le composé de formule
   (XX) obtenu ci-dessus à l'étape précédente, avec un composé de formule (XXI) dans lequel W F, I et n ont les mêmes significations que celles indiquées précédemment pour la sous-unité B3 de formule (VI), pour conduire à un composé de formule (XXII) ;
5) dans une cinquième étape, on déprotège complètement, dans un solvant organique tel que par exemple du dichlorométhane anhydre, le composé de formule (XXII) obtenu ci-dessus à l'étape précédente, en présence de pipéridine puis de fluorure de tetrabutylammonium pour conduire à un composé de formule (XXIII);
6) dans une sixième étape, on fait réagir, dans un solvant organique tel que par exemple le DMF anhydre, le composé de formule (XXIII) obtenu ci-dessus à l'étape précédente, avec un composé F, respectivement I, préalablement fonctionnalisé par un groupement NHS, pour conduire à un composé de formule (XXIV) (composé de type (I-2)).

Les procédés P1 et P2 représentés sur les schémas D et E ci-dessus comprennent un nombre minimal d'étapes. Il doit toutefois être bien entendu que suivant la nature du substrat enzymatique de formule (I-1) ou (I-2) que l'on souhaite obtenir, des réactions supplémentaires de protection/déprotection peuvent être nécessaires, en particulier si l'on souhaite appliquer les procédés P1, respectivement P2, à des substrats enzymatiques de formule (I-1), respectivement (I-2), dans lesquels F et I sont différents. Ces réactions sont réalisées de façon classique, selon les méthodes connues de l'homme du métier.

A titre d'exemple supplémentaire, les composés de formule (I-1) dans lesquels F et I sont différents et X = NH, peuvent notamment être préparés selon le procédé P3, tel que représenté sur le schéma F suivant : 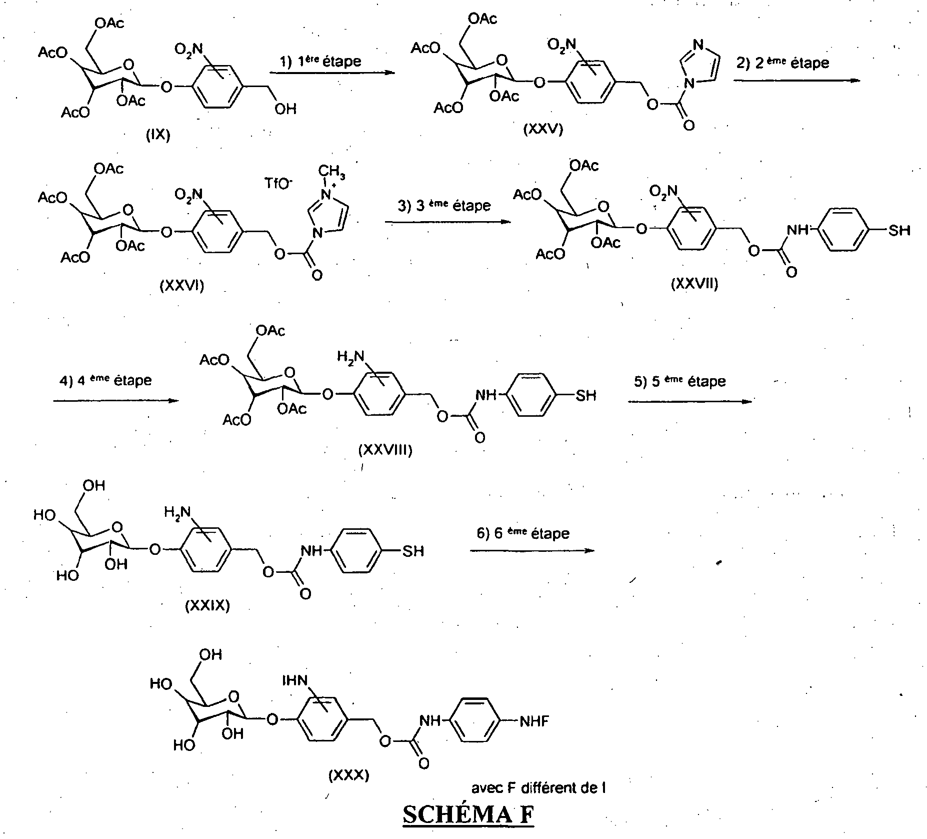 selon lequel :
1) dans une première étape, on fait réagir, dans un solvant organique tel que par exemple le dichlorométhane, un composé de formule (IX) tel que défini à l'étape 2) du procédé P1 avec de la 4-(diméthylamino)pyridine (DMAP), en présence de carbonyle diimidazole pour conduire à un composé de formule (XXV) dans lequel "Ac" signifie acétyle ;
2) dans une deuxième étape, on fait réagir, dans un solvant organique tel que par exemple le dichlorométhane, le composé de formule (XXV) obtenu ci-dessus à l'étape précédente avec du méthyl trifluorométhanesulfonate pour conduire à un composé de formule (XXVI) ;
3) dans une troisième étape, on fait réagir, dans un solvant organique tel que par exemple le dichlorométhane, le composé de formule (XXVI) obtenu ci-dessus à l'étape précédente avec 4-aminothiophénol pour conduire à un composé de formule (XXVII) ;
4) dans une quatrième étape, on effectue, dans un solvant organique tel que par exemple le méthanol, une réduction du composé de formule (XXVII) obtenu ci-dessus à l'étape précédente, en présence d'un agent réducteur pour conduire à un composé de formule (XXVIII) ;
5) dans une cinquième étape, on hydrolyse, dans un solvant organique tel que le méthanol, les groupements acétyle du composé de formule
   (XXVIII) en présence par exemple de méthylate de sodium, pour conduire à un composé de formule (XXIX) ; et
6) dans une sixième étape, on fait réagir, dans un solvant organique tel que par exemple du diméthylformamide anhydre, le composé de formule (XXIX) obtenu ci-dessus à l'étape précédente, avec un composé fluorophore préalablement fonctionnalisé par un groupement maléimide et un composé inhibiteur de la fluorescence du groupement fluorophore, ledit composé inhibiteur ayant préalablement été fonctionnalisé par un groupement NHS, en présence de diéthylamine, pour conduire à un composé de formule (XXX) dans lequel F est différent de I (composé de type (I-1)).

A chaque étape de ces procédés P1, P2 et P3, les composés intermédiaires, et le(s) composé(s) final (finaux) en fin de synthèse, sont de préférence lavés, isolés et purifiés selon les méthodes classiquement utilisées à cet effet telles que par exemple la purification sur colonne de gel de silice.

Ainsi que cela a été amplement décrit et explicité ci-avant, les substrats enzymatiques de formule (I) conformes à l'Invention peuvent être utilisés pour la détection d'une activité enzymatique *in vitro* et *in vivo.*

La présente Invention a donc pour deuxième objet, l'utilisation d'au moins un substrat enzymatique de formule (I) tel que défini précédemment, à titre de réactif fluorescent pour la détection d'une activité enzymatique *in vitro.*

Selon une forme de réalisation particulièrement préférée, le groupement fluorophore F des substrats enzymatiques de formule (I) est choisi parmi les groupements absorbant et émettant dans le proche infrarouge, en particulier entre 640 et 900 nm, afin de permettre une utilisation *in vivo.* Dans ce cas, la présente Invention a également pour objet, l'utilisation d'au moins un substrat enzymatique de formule (I) dans lequel le groupement fluorophore F est choisi parmi les groupements absorbant et émettant dans le proche infrarouge, pour la préparation d'un réactif de diagnostic destiné à l'imagerie fonctionnelle *in vivo* et en particulier pour imager, par fluorescence, l'expression des gènes rapporteurs *lacZ* et *gusA* de *E. coli.*

Enfin l'Invention a pour objet un réactif de diagnostic caractérisé par le fait qu'il comprend au moins une solution constituée d'eau ou d'un mélange d'eau et d'au moins un solvant organique, ladite solution renfermant au moins un substrat enzymatique de formule (I) tel que défini précédemment.

Selon une forme de réalisation particulière et préférée de l'invention, le réactif est un réactif de diagnostic *in vivo* et le substrat enzymatique de formule (I) comprend au moins un groupement fluorophore F choisi parmi les groupements fluorophores absorbant et émettant dans le proche infrarouge.

A titre de solvants organiques utilisables, on peut citer les solvants classiquement utilisés pour la préparation de réactifs de diagnostic parmi lesquels figurent par exemple les alcools inférieurs tels que l'éthanol et le diméthylsulfoxyde (DMSO). Lorsque qu'ils sont utilisés, ces solvants peuvent représenter jusqu'à 50 % (en volume) de la solution renfermant le substrat enzymatique de formule (I).

Selon une forme de réalisation particulière de l'Invention, la solution peut en outre renfermer un tampon physiologiquement acceptable tel qu'un tampon phosphate comme par exemple du PBS ("Phosphate Buffer Saline") à pH 7,2.

Au sein du réactif de diagnostic conforme à l'Invention, la concentration du ou des substrats enzymatiques de formule (I) est de préférence comprise entre 1 µM et 1 mM environ, plus préférentiellement entre 10 µM et 200 µM environ. Selon une forme de réalisation particulièrement préférée de l'Invention, cette concentration est de 100 µM environ.

Outre les dispositions qui précèdent, l'Invention comprend encore d'autres dispositions qui ressortiront du complément de description qui suit, qui se rapporte à des exemples de préparation des exemples de synthèse de substrats enzymatiques de formule (I), ainsi qu'aux dessins annexés dans lesquels :

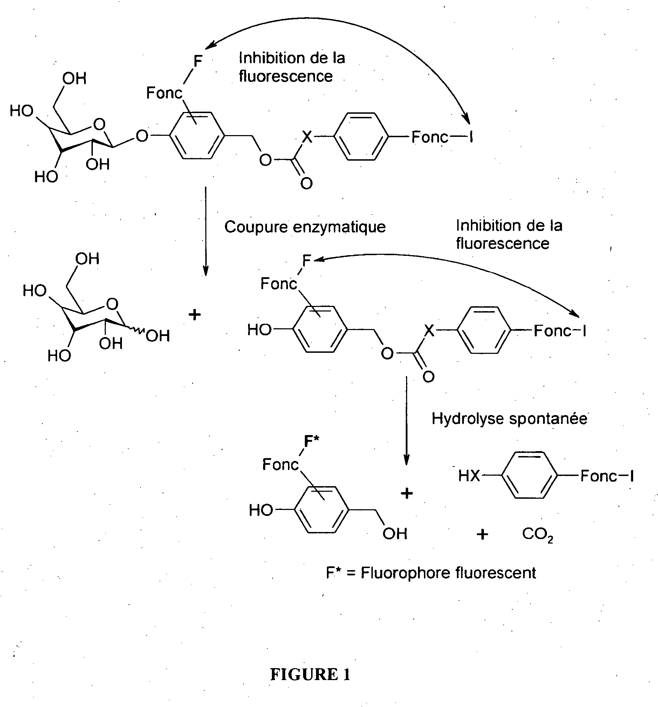

- la figure 1 illustre le principe de fonctionnement d'un substrat enzymatique de type (I-1). Sur cette figure, F, I, Fonc et X sont tels que définis précédemment ; Chacun des deux groupes F et I est greffé sur des noyaux aromatiques distincts de part et d'autre des fonctions carbonate ou carbamate, les deux positions pouvant néanmoins être interchangées. Le clivage enzymatique libère le bras autosécable du sucre qui s'hydrolyse ensuite spontanément en deux sous unités distinctes, chacune portant l'un des deux groupes F ou I. Les deux groupes F et I étant dès lors distants, le groupe F peut émettre de la fluorescence.

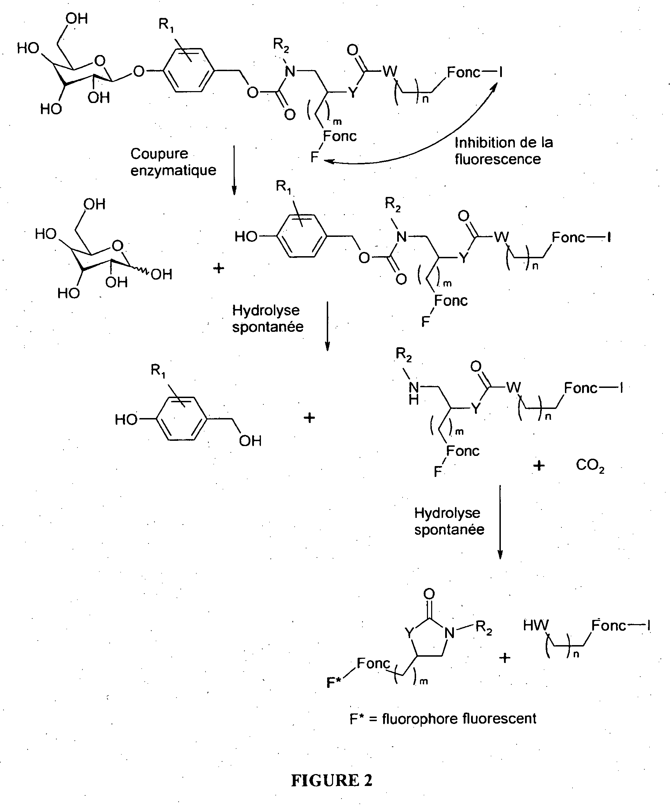

- la figure 2 illustre le principe de fonctionnement d'un substrat enzymatique de (I-2). Sur cette figure, F, I, Fonc, R₁, R₂, Y, W, m, et n sont tels que définis précédemment. Chacun des deux groupes F et I est greffé de part et d'autre des fonctions carbonate, carbamate ou urée. Les deux positions pouvant néanmoins être interchangées. Le clivage enzymatique libère le bras autosécable du sucre qui s'hydrolyse spontanément en deux sous unités, l'une aromatique et l'autre comportant encore les deux groupes F et I. Une ultime réaction intramoléculaire spontanée engendre la séparation des deux groupes F et I au niveau de la fonction carbonate, amide ou encore urée. Les deux groupes F et I étant dès lors distants, le groupe F peut alors émettre de la fluorescence.

Il doit être bien entendu, toutefois, que les exemples qui suivent ne sont donnés qu'à titre d'illustration de l'Invention dont ils ne constituent par ailleurs, en aucun cas, une limitation.

### EXEMPLE 1 : SYNTHÈSE D'UN SUBSTRAT ENZYMATIQUE CONFORME A L'INVENTION DANS LEQUEL F = I = CY5, ET X = O (Composé de formule (I-1)

Dans cet exemple, on a synthétisé le composé (7) suivant :

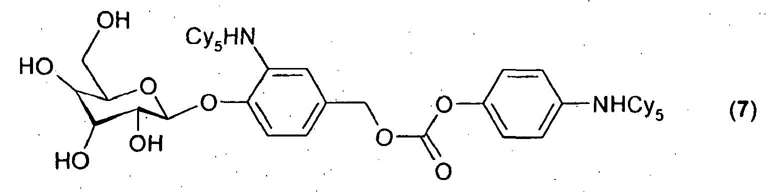

### 1) Première étape : Synthèse de l'intermédiaire (1)

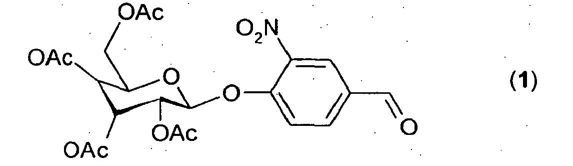

A une solution d'α-D-acétobromogalactose (10 g, 24,3 mmol) dans de l'acétonitrile anhydre (100 ml) ont été additionnés 6,9 g (41,3 mmol) de 4-hydroxy-3-nitrobenzaldéhyde et 19,7 g (85,1 mmol) d'oxyde d'argent. Le mélange réactionnel a été agité à température ambiante, sous atmosphère d'argon et à l'abri de la lumière, pendant 12 h. Puis, le mélange a été filtré sur silice et le filtrat a été concentré sous pression réduite. On a ainsi obtenu 7,44 g de l'intermédiaire (**1**) sous la forme d'un solide jaune pâle (rendement : 62 %). Le produit a ensuite été directement engagé dans l'étape suivante, sans purification supplémentaire.

### 2) Deuxième étape : Synthèse de l'intermédiaire (2)

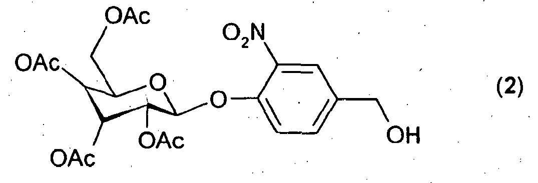

A une solution de 7,44 g (14.9 mmol) de l'intermédiaire **(2)** obtenu ci-dessus à l'étape précédente, dans 160 ml d'un mélange trichlorométhane/isopropanol (CHCl₃/iPrOH : 3/1, v/v), et refroidie à 0°C sous atmosphère d'argon, on a additionné 6,8 g (180 mmol) de borohydrure de sodium par petites portions. Après 12 h d'agitation à température ambiante, le mélange réactionnel a été hydrolysé par une solution saturée d'hydrogénocarbonate de sodium. La phase aqueuse a été extraite deux fois avec 300 ml dichlorométhane ; la phase organique obtenue a été lavée deux fois avec 200 ml l'eau, séchée sur sulfate de magnésium, filtrée sur fritté puis concentrée sous pression réduite. On a ainsi obtenu 5,86 g de l'intermédiaire (**2**) attendu sous la forme d'un solide jaune pâle (rendement : 78%). Le produit a ensuite été directement engagé dans l'étape suivante, sans purification supplémentaire.

### 3) Troisième étape : Synthèse de l'intermédiaire (3)

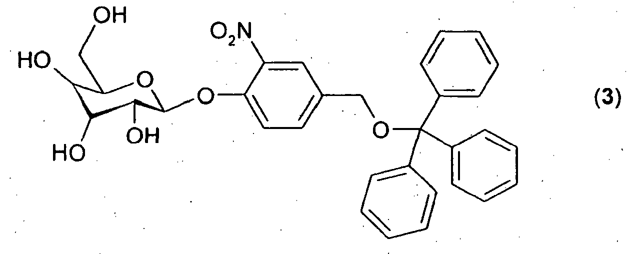

A une solution de 1,03 g (2,05 mmol) de l'intermédiaire (2) obtenu ci-dessus à l'étape précédente, dans 20 ml de dichlorométhane, on a ajouté 1,72 g (6,17 mmol) de chlorure de trityle, 860 µl (6,17 mmol) de triéthylamine et 25 mg (0,205 mmol) de 4-(diméthylamino)pyridine (DMAP). Le mélange réactionnel a été agité pendant 12 h à température ambiante avant d'être purifié directement par chromatographie sur gel de silice en utilisant comme phase liquide un mélange acétate d'éthyle/heptane/triéthylamine (AcOEt/Heptane/Et₃N: 20/79/1, v/v/v). On a ainsi obtenu 1,18 g d'un solide beige (rendement : 77%).

A une solution de 1,18 g (1,59 mmol) de ce produit dans 16 ml de méthanol anhydre, on a ajouté 86 mg (1,59 mmol) de méthylate de sodium. Le mélange réactionnel a été agité à température ambiante pendant 12 h avant d'être acidifié jusqu'à pH = 5 avec de la résine Dowex® H⁺. Après filtration puis concentration sous pression réduite, on a obtenu 839 mg de l'intermédiaire (**3**) sous la forme d'un solide jaune (rendement : 92%). Le produit a ensuite été directement engagé dans l'étape suivante, sans purification supplémentaire.

### 4) Quatrième étape : Synthèse de l'intermédiaire (4)

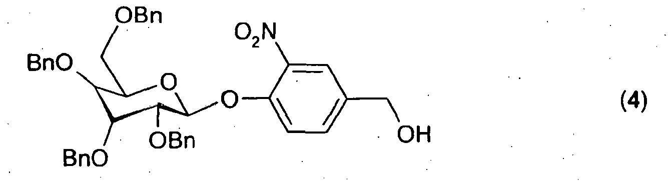

dans lequel "Bn" signifie benzyle.

A une solution refroidie à 0°C sous atmosphère d'argon de 839 mg (1,46 mmol) de l'intermédiaire **(4)** obtenu ci-dessus à l'étape précédente, dans 15 ml de diméthylformamide (DMF) anhydre, on a ajouté 351 mg (8,77 mmol) d'hydrure de sodium à 60 %. Après 10 minutes d'agitation, on a ensuite ajouté 1,39 ml (11,7 mmol) de bromure de benzyle. Le mélange réactionnel a été agité à température ambiante pendant 12 h avant d'être hydrolysé. Après une partition H₂O/AcOEt, la phase aqueuse a été extraite deux fois avec 150 ml d'acétate d'éthyle ; la phase organique obtenue a été lavée deux fois avec 100 ml d'une solution aqueuse saturée de chlorure de sodium, séchée sur sulfate de magnésium, filtrée sur fritté puis concentrée sous pression réduite. Le résidu a été purifié par chromatographie sur gel de silice en utilisant comme phase liquide un mélange AcOEt/Heptane/Et₃N : 10/89/1, v/v/v. On a obtenu 888 mg d'une huile jaune (rendement : 65%).

A une solution refroidie à 0°C sous atmosphère d'argon de 888 mg (0,951 mmol) de ce produit dans 5 ml de dichlorométhane, on a ensuite ajouté 71 µl (0,951 mmol) d'acide trifluoroacétique. Le mélange réactionnel a été agité à 0°C pendant 1 h avant d'être neutralisé par une solution aqueuse d'hydrogénocarbonate de sodium. La phase aqueuse a été extraite deux fois avec 150 ml de dichlorométhane ; la phase organique obtenue a été lavée deux fois avec 100 ml d'eau, séchée sur sulfate de magnésium, filtrée sur fritté puis concentrée sous pression réduite. Le résidu ainsi obtenu a été purifié par chromatographie sur gel de silice (AcOEt/Heptane : 40/60, v/v) pour conduire à 605 mg de l'intermédiaire **(4)** attendu sous la forme d'huile jaune pâle (rendement : 92%).

### 5) Cinquième étape : Synthèse de l'intermédiaire (5)

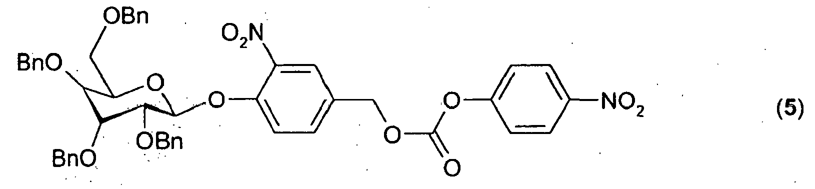

A une solution de 233 mg (0,337 mmol) de l'intermédiaire **(4)** obtenu ci-dessus à l'étape précédente dans 2 ml de dichlorométhane, on a ajouté 94 µl (0,674 mmol) de triéthylamine et 102 mg (0,505 mmol) de 4-nitrophényl-chloroformate. Le mélange réactionnel a été agité à température ambiante pendant 12 h avant d'être hydrolysé avec une solution aqueuse d'hydrogénocarbonate de sodium. La phase aqueuse a été extraite deux fois avec 50 ml de dichlorométhane ; la phase organique obtenue a été lavée deux fois avec 25 ml d'eau, séchée sur sulfate de magnésium, filtrée sur fritté puis concentrée sous pression réduite. Le résidu a été purifié par chromatographie sur gel de silice (AcOEt/Heptane : 15/85, v/v) pour conduire à 213 mg de l'intermédiaire (**5**) attendu sous la forme d'une huile jaune (rendement : 74%).

### 6) Sixième étape : Synthèse de l'intermédiaire (6)

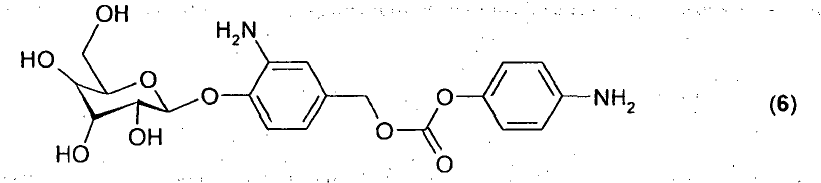

A une solution de 165 mg (0,192 mmol) de l'intermédiaire **(5)** obtenu ci-dessus à l'étape précédente, dans 2 ml de méthanol, on a ajouté 17 mg de palladium sur charbon à 10% d'eau. Le mélange réactionnel a été purgé avec du dihydrogène cinq fois avant d'être agité à température ambiante pendant 12 h. Après filtration sur célite et concentration sous pression réduite, on a obtenu 78 mg du composé intermédiaire (**6**) sous la forme d'une huile jaune (rendement : 93%). Le produit a ensuite été directement engagé dans l'étape suivante, sans purification supplémentaire.

### 7) Septième étape : Synthèse du composé (7) conforme à l'invention

A une solution de 5 mg (5,7 µmol) de Cy5-NHS (GE-Amersham) dans 500 µl de DMF anhydre sous atmosphère d'argon, on a ajouté 250 µl de diéthylamine et 1,3 mg (2,93 µmol) de l'intermédiaire **(6)** obtenu ci-dessus à l'étape précédente. Le mélange réactionnel a été agité à température ambiante pendant 12 h avant d'être concentré sous pression réduite. Le substrat enzymatique **(7)** attendu a été obtenu après purification par HPLC avec un rendement de 72 %.

### EXEMPLE 2 : SYNTHÈSE D'UN SUBSTRAT ENZYMATIQUE CONFORME A L'INVENTION DANS LEQUEL F = I = Cy5, ET X = Y = O, m = n = 1 (Composé de formule (1-2)

Dans cet exemple, on a synthétisé le composé **(13)** suivant :

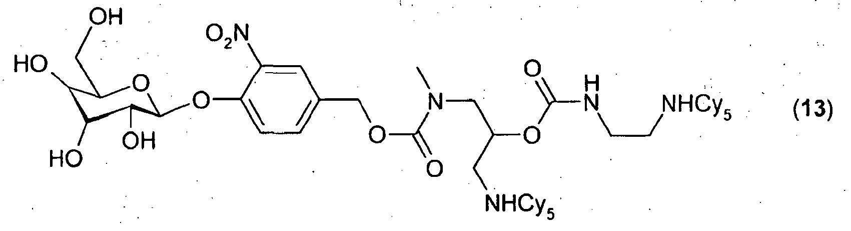

### 1) Première étape : Synthèse de l'intermédiaire (8)

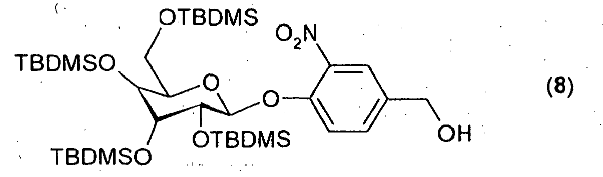

dans lequel "TBDMS" représente le groupement protecteur *tert*butyldiméthylsilane.

A une solution de 1,21 g (2,11 mmol) de l'intermédiaire **(3)** tel qu'obtenu ci-dessus à l'issue de la troisième étape de l'exemple 1, dans 11 ml de dichlorométhane, on a ajouté 862 mg (12,7 mmol) d'imidazole et 1,9 g (12,7 mmol) de *tert*butyldiméthylchlorosilane. Le mélange réactionnel a été agité à température ambiante pendant 12 h avant d'être hydrolysé avec une solution aqueuse saturée d'hydrogénocarbonate de sodium. La phase aqueuse a été extraite deux fois avec 150 ml de dichlorométhane ; la phase organique obtenue a été lavée deux fois avec 100 ml d'eau, séchée sur sulfate de magnésium, filtrée sur fritté puis concentrée sous pression réduite. Le résidu a été purifié par chromatographie sur gel de silice (AcOEt/Heptane : 20/80, v/v) pour conduire à 1,57 g d'un produit se présentant sous la forme d'une huile jaune (rendement : 72%).

A une solution refroidie à 0°C sous atmosphère d'argon de 1,57 g (1,52 mmol) de ce produit dans 8 ml de dichlorométhane, on a ajouté 113 µl (1,52 mmol) d'acide trifluoroacétique. Le mélange réactionnel a été agité à 0°C pendant 1 h avant d'être neutralisé par une solution aqueuse d'hydrogénocarbonate de sodium. La phase aqueuse a été extraite deux fois avec 150 ml de dichlorométhane ; la phase organique obtenue a été lavée deux fois avec 100 ml d'eau, séchée sur sulfate de magnésium, filtrée sur fritté puis concentrée sous pression réduite. Le résidu a été purifié par chromatographie sur gel de silice (AcOEt/Heptane : 40/60, v/v). 1,05 g d'huile jaune sont obtenus (rendement : 88%).

### 2) Deuxième étape : Synthèse de l'intermédiaire (9)

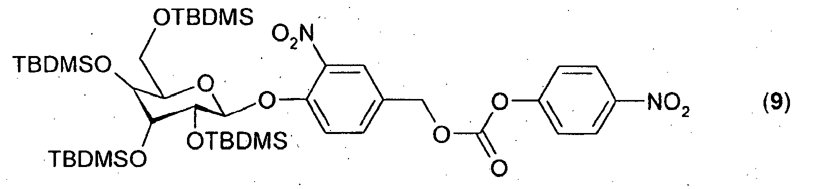

A une solution de 1,0 g (1,27 mmol) de l'intermédiaire **(8)**, obtenu ci-dessus à l'étape précédente, dans 6 ml de dichlorométhane, on a ajouté 354 µl (2,54 mmol) de triéthylamine et 384 g (1,90 mmol) de 4-nitrophényl-chloroformate. Le mélange réactionnel a été agité à température ambiante pendant 12 h avant d'être hydrolysé avec une solution aqueuse d'hydrogénocarbonate de sodium. La phase aqueuse a été extraite deux fois avec 150 ml de dichlorométhane ; la phase organique obtenue a été lavée deux fois avec 100 ml d'eau, séchée sur sulfate de magnésium, filtrée sur fritté puis concentrée sous pression réduite. Le résidu a été purifié par chromatographie sur gel de silice (AcOEt/Heptane : 15/85, v/v) pour conduire à 919 mg du composé intermédiaire (**9**) sous forme d'une huile jaune (rendement : 76%).

### 3) Troisième étape : Synthèse de l'intermédiaire (10) (X = O, m = 1)

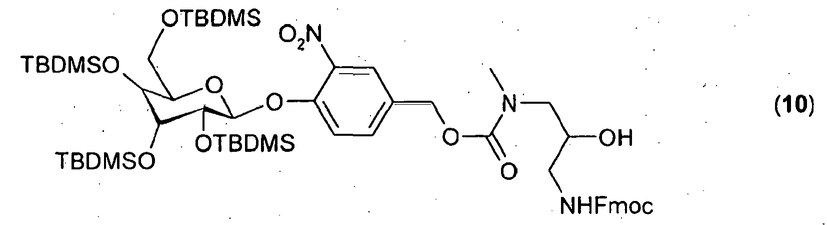

dans lequel Fmoc est le groupement protecteur fluorénylméthyloxycarbonyle. A une solution de 278 mg (0,295 mmol) de l'intermédiaire **(9)** obtenu ci-dessus à l'étape précédente, dans 1,5 ml de dichlorométhane, on a ajouté 114 mg (0,350 mmol) de (9H-fluoren-9-yl)méthyl-2-hydroxy-3-(méthylamino)propylcarbamate. Après 12 h d'agitation à température ambiante, le mélange réactionnel a été hydrolysé par une solution saturée d'hydrogénocarbonate de sodium. La phase aqueuse a été extraite deux fois avec 50 ml de dichlorométhane ; la phase organique obtenue a été lavée deux fois avec 30 ml d'eau, séchée sur sulfate de magnésium, filtrée sur fritté puis concentrée sous pression réduite. On a obtenu 226 mg de composé intermédiaire (**10**) sous la forme d'une huile jaune (rendement : 68%). Le produit a ensuite été directement engagé dans l'étape suivante, sans purification supplémentaire.

### 4) Quatrième étape : Synthèse de l'intermédiaire 11 (X = O, Y = N, B = Cy5, m = n = 1)

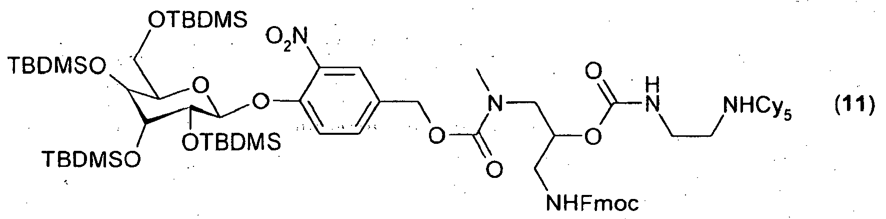

A une solution refroidie à 0°C sous atmosphère d'argon de 212 mg (0,186 mmol) du composé intermédiaire **(10)**, obtenu ci-dessus à l'étape précédente, dans 2 ml de dichlorométhane anhydre, on a ajouté 22 mg (0,223 mmol) de phosgène. Le mélange réactionnel a été agité pendant 1 h à 0°C avant d'ajouter 165 mg (0,372 mmol) du composé de formule suivante : H₂N-(CH₃)₂-NHCy5. Le mélange réactionnel a été agité à température ambiante pendant 2 h avant d'être concentré sous pression réduite. On a obtenu 14 mg d'un résidu qui a ensuite été purifié par HPLC pour donner 11,3 mg du composé intermédiaire **(11)** attendu.

### 5) Cinquième étape : Synthèse de l'intermédiaire (12) (X = O, Y = N, B = Cy5, m = n =1)

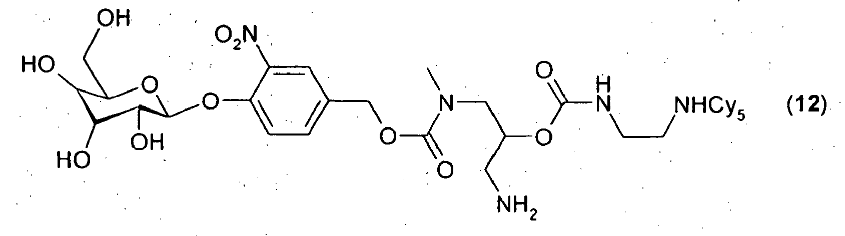

A une solution de 11,3 mg (5,9 µmol) du composé intermédiaire (**11**), obtenu ci-dessus à l'étape précédente, dans 1 ml de dichlorométhane anhydre, on a ajouté 1,16 µl (11,8 µmol) de pipéridine. Le mélange réactionnel a été agité pendant 2 h avant puis on a ajouté 23,6 µl (23,6 µmol) d'une solution de fluorure de tétrabutylammonium à 1.0 M dans le tetrahydrofurane (THF). Le mélange réactionnel a été agité pendant 4 h avant d'être concentré sous pression réduite. Le résidu a été purifié par HPLC pour conduire à 8,7 mg du composé intermédiaire **(12)**.

### 6) Sixième étape : Synthèse du composé (13)

A une solution de 6,7 mg (7,6 µmol) de Cy5-NHS dans 500 µl de DMF anhydre, sous atmosphère d'argon, on a ajouté 250 µl de diéthylamine et 4,8 mg (3,8 µmol) du composé intermédiaire (**12**) obtenu ci-dessus à l'étape précédente. Le mélange réactionnel a été agité à température ambiante pendant 12 h avant d'être concentré sous pression réduite. Après purification par HPLC, on a obtenu 4,4 mg du composé de formule (**13**) attendu.

### EXEMPLE 3 : SYNTHÈSE D'UN SUBSTRAT ENZYMATIQUE CONFORME A L'INVENTION DANS LEQUEL F = Cy5, I = Cy7Q ET X = NH (Composé de formule (I-1)

Dans cet exemple, on a synthétisé le composé **(105)** suivant :

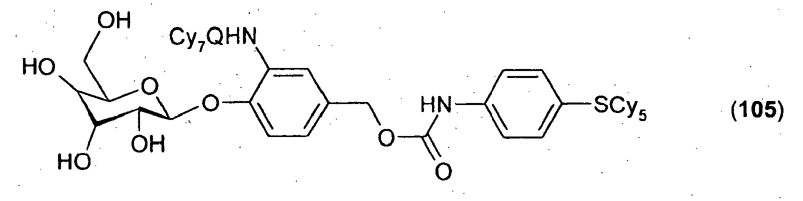

### 1) Première étape : Synthèse de l'intermédiaire (100)

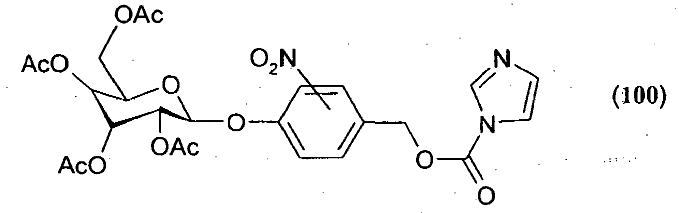

A une solution de l'intermédiaire **(2)** tel qu'obtenu ci-dessus à l'issue de la deuxième étape de l'exemple 1 (1,0 g, 2,0 mmol) dans 15 ml de dichlorométhane, on a additionné 49 mg (0,4 mmol) de DMAP et 650 mg (4,0 mmol) de carbonyle diimidazole. Le mélange réactionnel a été agité à température ambiante, pendant 12 h avant d'être concentré sous pression réduite. L'huile obtenue a été purifiée par chromatographie sur gel de silice (AcOEt/Heptane : 50/50, v/v puis 70/30, v/v et 100/0, v/v). On a ainsi obtenu 702 mg de l'intermédiaire **(100)** sous la forme d'une mousse blanche (rendement : 59 %).

### 2) Deuxième étape : Synthèse de l'intermédiaire (101)

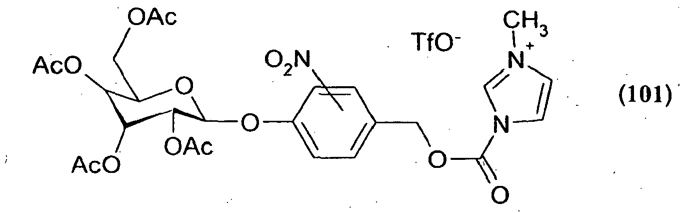

A une solution de 700 mg (1,18 mmol) de l'intermédiaire **(100)** obtenu ci-dessus à l'étape précédente, dans 8 ml de dichlorométhane anhydre, et sous atmosphère d'argon, on a additionné 267 µl (2,36 mmol) de méthyl trifluorométhanesulfonate. Le mélange réactionnel a été agité pendant 10 min à température ambiante, avant d'être concentré sous pression réduite. Le résidu a été trituré plusieurs fois dans l'éther diéthylique avant d'être séché sous vide. On a ainsi obtenu 822 mg de l'intermédiaire **(101)** attendu sous la forme d'un solide blanc (rendement : 92%). Le produit a ensuite été directement engagé dans l'étape suivante, sans purification supplémentaire.

### 3) Troisième étape : Synthèse de l'intermédiaire (102)

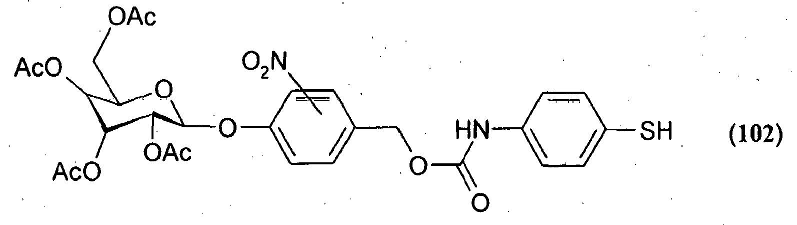

A une solution de 700 mg (0,924 mmol) de l'intermédiaire **(101)** obtenu ci-dessus à l'étape précédente, dans 5 ml de dichlorométhane anhydre et sous atmosphère d'argon, on a ajouté 231 mg (1,85 mmol) de 4-aminothiophénol. Le mélange réactionnel a été agité pendant 2 h à température ambiante avant d'être concentré sous pression réduite. L'huile obtenue a été purifié par chromatographie sur gel de silice (AcOEt/Heptane : 30/70, v/v). On a ainsi obtenu 457 mg du composé intermédiaire (**102**) attendu sous la forme d'un solide jaune pâle (rendement : 76%).

### 4) Quatrième étape : Synthèse de l'intermédiaire (103)

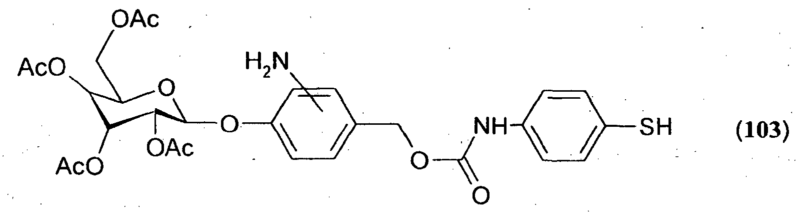

A une solution de 330 mg (0,507 mmol) de l'intermédiaire **(102)** obtenu ci-dessus à l'étape précédente, dans 3 ml de méthanol et 500 µl d'acide chlorhydrique concentré, on a ajouté 192 mg (1,01 mmol) de dichlorure d'étain. Le mélange réactionnel a ensuite été agité pendant 12 heures à température ambiante. Après une partition dichlorométhane/eau, le pH du mélange a été relevé à 8-9 en ajoutant une solution aqueuse saturée d'hydrogénocarbonate de sodium. La phase aqueuse a été extraite deux fois avec du dichlorométhane, séchée sur sulfate de magnésium, filtrée sur fritté puis concentrée sous pression réduite. Le résidu a été purifié par chromatographie sur gel de silice (AcOEt/Heptane : 70/30, v/v). On a obtenu 226 mg de l'intermédiaire **(103)** attendu sous la forme d'une huile jaune (rendement : 72%).

### 5) Cinquième étape : Synthèse de l'intermédiaire (104)

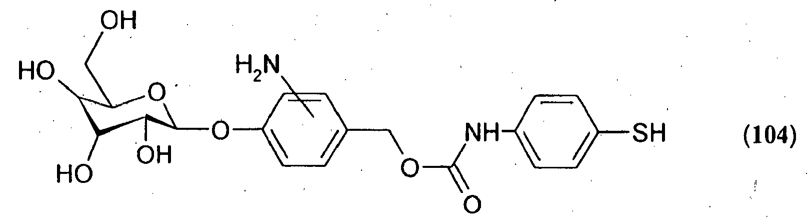

A une solution de 210 mg (0,338 mmol) de l'intermédiaire **(103)** obtenu ci-dessus à l'étape précédente dans 3 ml de méthanol anhydre, on a ajouté 18 mg (0,338 mmol) de méthylate de sodium. Le mélange réactionnel a été agité à température ambiante pendant 12 h avant d'être acidifié jusqu'à pH 7 avec de la résine Dowex ® H⁺. Après filtration puis concentration sous pression réduite, on a obtenu 145 mg de l'intermédiaire **(104)** attendu sous la forme d'un solide jaune (rendement 95%). Le produit a ensuite été engagé dans l'étape suivante sans purification supplémentaire.

### 6) Sixième étape : Synthèse du composé (105) conforme à l'Invention

A une solution de 24 mg (26,5 µmol) de Cy7Q-NHS et de 20 mg (26,5 µmol) de Cy5-maléimide dans 1 ml de DMF anhydre et sous atmosphère d'argon, on a ajouté 500 µl de diéthylamine et 10 mg (22,1 µmol) d'intermédiaire **(104)** obtenu ci-dessus à l'étape précédente. Le mélange réactionnel a été agité à température ambiante pendant 12 h avant d'être concentré sous pression réduite. Après purification par HPLC préparative, on a obtenu 26 mg du composé **(105)** attendu (rendement : 62%).

### EXEMPLE 4 : SYNTHÈSE D'UN SUBSTRAT ENZYMATIQUE CONFORME A L'INVENTION DANS LEQUEL F = Cy5,I = FluoQuench 661, R₁ = NO₂, R₂ = H, Y = O, W = N, et m = n = 1

### (Composé de formule (I-2))

Dans cet exemple, on a synthétisé le composé (**200**) suivant :

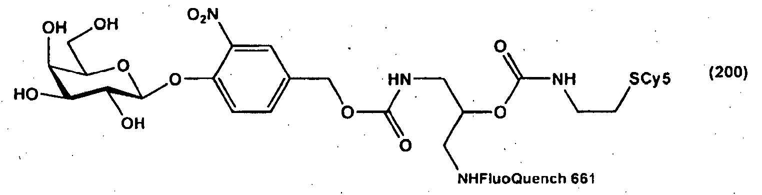

### 1) Première étape : Synthèse de l'intermédiaire (201)

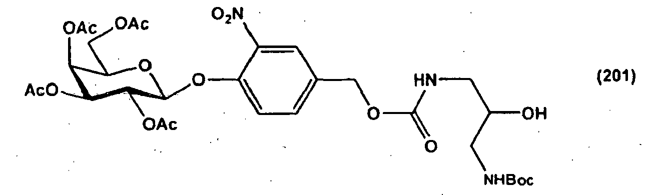

A une solution de 700 mg (0,924 mmol) de l'intermédiaire **(101)** tel qu'obtenu à l'issue de la deuxième étape de l'exemple 3, dans 5 mL du *N,N-*diméthylformamide anhydre et sous atmosphère d'argon, on a ajouté 351 mg (1,85 mmol) de *tert*-butyl-3-amino-2-hydroxypropyl carbamate. Le mélange réactionnel a été agité pendant 2 h à température ambiante avant d'être concentré sous pression réduite. L'huile obtenue a été purifiée par chromatographie sur gel de silice (AcOEt/Heptane : 60/40, v/v). On a ainsi obtenu 509 mg du composé intermédiaire (**201**) attendu sous la forme d'une huile jaune pâle (rendement : 77%).
RMN ¹H (CDCl₃, 200 MHz) :
δ (ppm) : 7,84 (d, J = 2 Hz, 1H); 7,58-7,30 (m, 1H); 7,40-7,35 (m, 1H); 5,95-5,89
(m, 1H), 5,64-5,50 (m, 3H); 5,17-5,08 (m, 4H); 4,31-4,10 (m, 4H); 3,85-3,79 (m,
1H); 3,36-3,21 (m, 3H); 2,23 (s, 3H); 2,17 (s, 3H); 2,12 (s, 3H); 2,09 (s, 3H); 1,48
(s, 9H); 1,30 (t, J = 7 Hz, 2H).
SM (ESI+) : 738,2 (M+Na)⁺, 1453,8 (2M+Na)⁺.

### 2) Deuxième étape : Synthèse de l'intermédiaire (202)

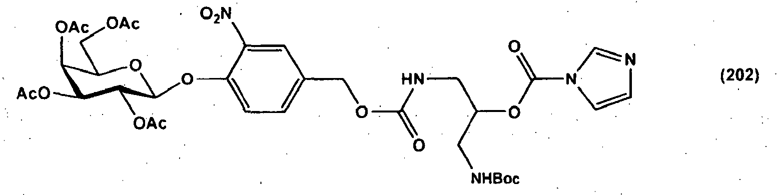

A une solution de 1,0 g (1,40 mmol) de l'intermédiaire (**201**) obtenu ci-dessus à l'étape précédente, dans 15 mL de dichlorométhane, on a additionné 45 mg (0,28 mmol) de DMAP et 454 mg (2,80 mmol) de carbonyle diimidazole. Le mélange réactionnel a été agité à température ambiante pendant 12 h avant d'être concentré sous pression réduite. L'huile obtenue a été purifiée par chromatographie sur gel de silice (AcOEt/Heptane : 80/20, v/v). On a ainsi obtenu 882 mg de l'intermédiaire (**202**) attendu sous la forme d'une mousse blanche (rendement : 78%).
RMN ¹H (CDCl₃, 200 MHz) :
δ (ppm) : 8,26 (d, J = 7 Hz, 1H); 7,83 (d, J = 2 Hz, 1H); 7,57-7,35 (m, 3H); 7,12
(s, 1H); 5,94-5,89 (m, 1H); 5,63-5,45 (m, 3H); 5,18-5,03 (m, 7H); 4,29-4,08 (m,
4H); 3,56-3,51 (m, 4H); 2,23 (s, 3H); 2,17 (s, 3H); 2,11 (s, 3H); 2,08 (s, 3H); 1,47 (s, 9H).
SM (ESI+) : 810,1 (M+H)⁺, 1618,8 (2M+H)⁺.

### 3) Troisième étape : Synthèse de l'intermédiaire (203)

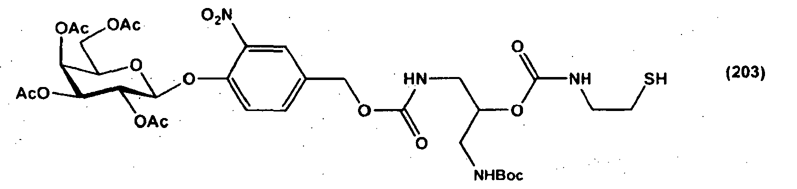

A une solution de 205 mg (0,253 mmol) de l'intermédiaire **(202)** obtenu ci-dessus à l'étape précédente, dans 2 mL de dichlorométhane anhydre et sous atmosphère d'argon, on a ajouté 353 µL (2,50 mmol) de triéthylamine et 58 mg (0,506 mmol) de chlorhydrate de cystéamine. Le mélange réactionnel a été agité pendant 12 h à température ambiante avant d'être concentré sous pression réduite. L'huile obtenue a été purifiée par chromatographie sur gel de silice (AcOEt/Heptane: 60/40, v/v). On a ainsi obtenu 130 mg du composé intermédiaire **(203)** attendu sous la forme d'une huile jaune pâle (rendement : 63%).
RMN ¹H (CDCl₃, 200 MHz) :
δ (ppm) : 7,84 (d, J = 2 Hz, 1H); 7,56 (dd, J = 2 et 9 Hz, 1H); 7,37 (d, J = 9 Hz,
1H); 5,73-5,66 (m, 1H); 5,63-5,48 (m, 2H); 5,19-5,08 (m, 4H); 4,99-4,91 (m, 1H);
4,80-4,72 (m, 1H); 4,34-4,07 (m, 3H); 3,54-3,24 (m, 4H); 2,92 (d, J = 7 Hz, 4H);
2,23 (s, 3H); 2,17 (s, 3H); 2,12 (s, 3H); 2,06 (s, 3H); 1,48 (s, 9H).
SM (ESI+) : 841,2 (M+Na)⁺; 1659,8 (2M+Na)⁺.

### 4) Quatrième étape : Synthèse de l'intermédiaire (204)

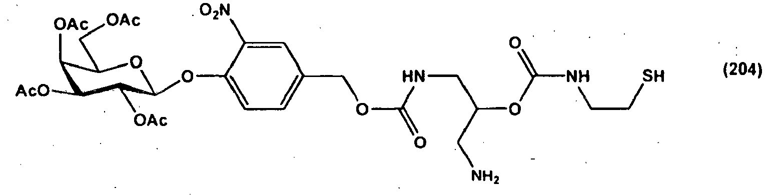

A une solution de refroidie à 0°C sous atmosphère d'argon de 210 mg (0,256 mmol) de l'intermédiaire **(203)** obtenu ci-dessus à l'étape précédente, dans 3 mL de dichlorométhane anhydre, on a ajouté 1 mL (13,0 mmol) d'acide trifluoroacétique. Le mélange réactionnel a été agité pendant 2 h à 0°C avant d'être hydrolysé avec une solution aqueuse d'hydrogénocarbonate de sodium. La phase aqueuse a été extraite deux fois avec 50 mL de dichlorométhane ; la phase organique obtenue a été lavée deux fois avec 50 mL d'eau, séchée sur sulfate de magnésium, filtrée sur fritté puis concentrée sous pression réduite. On a ainsi obtenu 117 mg du composé intermédiaire **(204)** attendu sous la forme d'une huile jaune pâle (rendement : 64%). Le produit a ensuite été directement engagé dans l'étape suivante, sans purification supplémentaire.
RMN ¹H (CDCl₃, 200 MHz) :
δ (ppm) : 7,83 (d, J = 2 Hz, 1H); 7,57 (dd, J = 2 et 9 Hz, 1H); 7,39 (d, J = 9 Hz,
1H); 5,63-5,50 (m, 2H); 5,34 (s, 3H, 5,19-5,08 (m, 4H); 4,82-4,76 (m, 1H); 4,31-
4,11 (m, 3H); 3,54-3,35 (m, 3H); 2,97-2,91 (m, 3H); 2,22 (s, 3H); 2,16 (s, 3H);
2,11 (s, 3H); 2,05 (s, 3H); 1,38-1,19 (m, 2H); 1,02-0,91 (m, 1H).
SM (ESI+) : 741,3 (M+Na)⁺; 1459,2 (2M+Na)⁺.

### 5) Cinquième étape : Synthèse de l'intermédiaire (205)

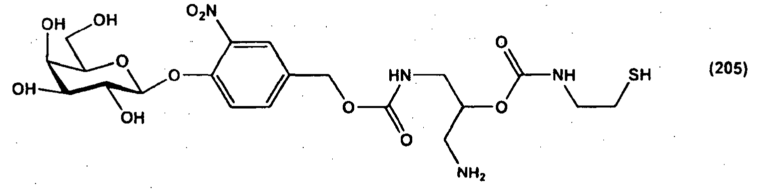

A une solution de 117 mg (0,163 mmol) de l'intermédiaire **(204)** obtenu ci-dessus à l'étape précédente, dans 2 mL de méthanol anhydre et sous atmosphère d'argon, on a ajouté 9 mg (0,163 mmol) de méthylate de sodium. Le mélange réactionnel a été agité pendant 12 h à température ambiante. Le pH de la solution est ensuite ajusté jusqu'à pH = 7 en ajoutant de la résine échangeuse d'ions H⁺/Na⁺. On a ainsi obtenu 45 mg du composé intermédiaire **(205)** attendu sous la forme d'un solide jaune pâle (rendement : 50%). Le produit a ensuite été directement engagé dans l'étape suivante, sans purification supplémentaire.
RMN ¹H (D₂O, 200 MHz) :
δ (ppm) : 7,84-7,22 (m, 3H) ; 5,09-4,87 (m, 4H) ; 3,89 (d, J = 3 Hz, 1H); 3,79-
3,61 (m, 6H); 3,30-3,05 (m, 4H) ; 2,67-2,62 (m, 4H).
SM (ESI+): 519,3 (M-S)⁺; 541,2 (M-S+Na)⁺; 550,5 (M+H)⁺; 573,3 (M+Na)⁺;
1099,4 (2M-2H)⁺.

### 6) Sixième étape : Synthèse de l'intermédiaire (206)

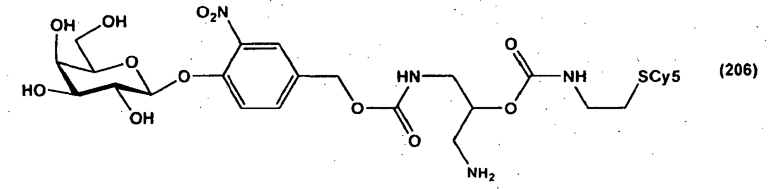

A une solution de 0,674 mg (1,22 µmol) de l'intermédiaire **(205)** obtenu ci-dessus à l'étape précédente, dans 150 µL de solution tampon PBS (pH = 7,4) à 0,01 mol.L⁻¹, on a ajouté 48 µL (24 µmol) d'une solution de tris(2-carboxyethyl)phosphine hydrochloride à 0,5 mol.L⁻¹. Le mélange réactionnel a été agité pendant 30 min à température ambiante avant d'additionner une solution de 1,0 mg (1,22 µmol) de Cy5 monomaléimide dans 50 µL de DMSO. Le mélange réactionnel a été agité pendant 12 h à température ambiante et à l'abri de la lumière avant d'être directement purifié par HPLC pour donner 0,77 mg du composé intermédiaire **(206)** attendu (rendement : 46%).
SM(ESI-) : 1327,4 (M-H)⁻.

### 7) Sixième étape : Synthèse du composé (200) conforme à l'Invention

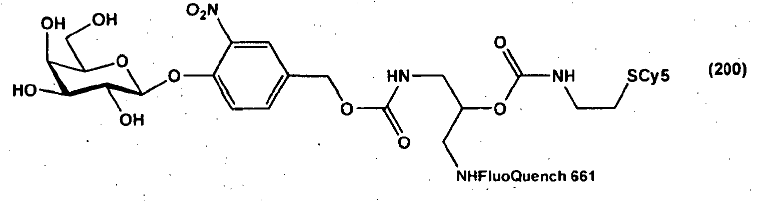

A une solution de 0,77 mg (0,561 µmol) de l'intermédiaire **(206)** obtenu ci-dessus à l'étape précédente, dans 50 µL de *N*,*N*-diméthylformamide, on a ajouté 5 µL (36 µmol) de triéthylamine et une solution de 1,0 mg (1,12 µmol) de FluoQuench 661 mono NHS ester dans 50 µL de *N*,*N*-diméthylformamide. Le mélange réactionnel a été agité pendant 12 h à température ambiante et à l'abri de la lumière avant d'être directement purifié par HPLC pour donner 0,52 mg du composé **(200)** conforme à l'Invention (rendement : 45%). SM(ESI-) : 2056,6 (M-H)⁻.

## Revendications

1. Substrat enzymatique fluorescent, **caractérisé par le fait qu'**il répond à la structure (I) suivante :
S-B(I)ₙ-F (I)
dans laquelle :
- S est un squelette de nature saccharidique constitué d'au moins une unité saccharidique et choisi parmi les monosaccharides, les oligosaccharides ayant de 2 à 9 unités saccharidiques et les polysaccharides ayant au moins 10 unités saccharidiques ;
- F est un groupement fluorophore porté par le bras espaceur B ;
- I est un groupement inhibiteur de la fluorescence de F ; ledit groupement I étant un substituant latéral d'au moins une sous-unité du bras espaceur B ;
étant entendu qu'aucune liaison covalente ne relie directement le groupement fluorophore au groupement inhibiteur.
- n est un nombre entier égal à 1 ou 2 ;
- B représente un bras espaceur autosécable constitué d'une ou de plusieurs sous-unités ; ledit bras B étant fixé en position anomérique de ladite unité saccharidique S, la liaison anomérique étant indifféremment en position α ou β, ledit bras B correspondant à un site reconnu et clivé par une enzyme et comprenant une sous-unité B 1 choisie parmi :
i) les groupements aromatiques monocycliques de formule (II) suivante : 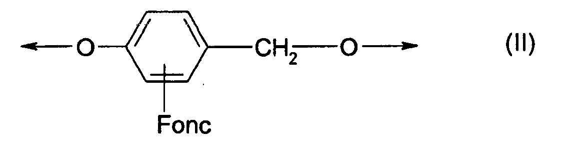 dans laquelle :
- Fonc est une fonction chimique réactive vis-à-vis d'une fonction chimique complémentaire d'un groupement fluorophore F ou respectivement d'un groupement inhibiteur de la fluorescence d'un groupement fluorophore F,
- la flèche partant de l'atome d'oxygène directement porté par le cycle phényle représente le point d'attachement de ladite sous-unité B1 avec une unité saccharidique du bras espaceur par l'intermédiaire d'une liaison covalente avec l'atome de carbone situé en position 1 anomérique de ladite unité saccharidique,
- la flèche partant de l'atome d'oxygène lié au radical -CH₂- représente le point d'attachement de ladite sous-unité B1 avec une sous-unité B2 choisie parmi les groupements aromatiques de formule (III) suivante : 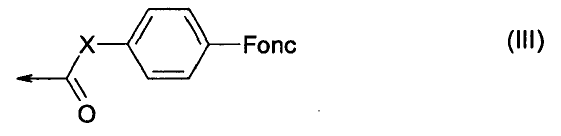 dans laquelle :
• Fonc est une fonction chimique réactive vis-à-vis d'une fonction chimique complémentaire d'un groupement inhibiteur de la fluorescence du groupement F ou respectivement d'un groupement fluorophore F ;
• la flèche représente le point d'attachement de ladite sous-unité B2 avec la sous-unité B1 par l'intermédiaire d'une liaison covalente avec l'atome d'oxygène de la sous-unité B1,
• X est O, NH ou S; et
ii) les groupements aromatiques monocycliques de formule (IV) suivante : 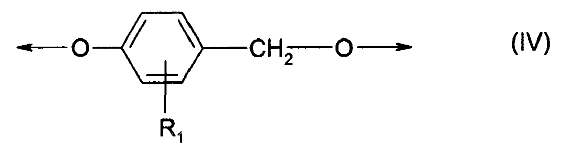 dans laquelle :
- R₁ est choisi parmi les groupements nitro, sulfate, amine et amine protégée par un groupement protecteur,
- la flèche partant de l'atome d'oxygène directement porté par l'atome de carbone du cycle phényle représente le point d'attachement de ladite sous-unité B1 avec une unité saccharidique du bras espaceur par l'intermédiaire d'une liaison covalente avec l'atome de carbone situé en position 1 anomérique de ladite unité saccharidique,
- la flèche partant de l'atome d'oxygène lié au radical -CH₂- représente le point d'attachement de ladite sous-unité B1 avec une sous-unité B2 choisie parmi les groupements de formule (V) suivante : 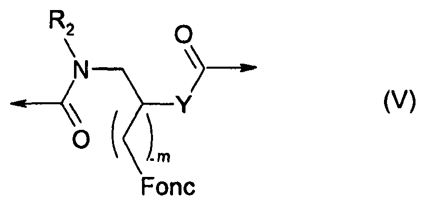 dans laquelle :
• R₂ est un atome d'hydrogène ou un radical alkyle en C₁-C₄,
• Fonc est une fonction chimique réactive vis-à-vis d'une fonction chimique complémentaire d'un groupement fluorophore F ou respectivement d'un groupement inhibiteur de la fluorescence d'un groupement fluorophore F ;
• m est un nombre entier allant de 1 à 10 ;
• Y est O, NH ou S ;
• les flèches représentent le point d'attachement de ladite sous-unité B2 avec d'une part la sous-unité B1 et d'autre part avec un atome d'azote ou d'oxygène porté par une sous-unité B3 de formule (VI) suivante : 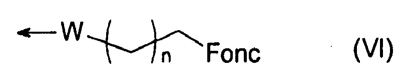
dans laquelle :
• W représente O, NH ou S ;
• la flèche représente le point d'attachement de l'atome d'azote, de soufre ou d'oxygène désigné pour W, par l'intermédiaire d'une liaison covalente avec un atome de carbone de la sous-unité B2,
• n est un nombre entier allant de 1 à 10 ;
• Fonc est une fonction chimique réactive vis-à-vis d'une fonction chimique complémentaire d'un groupement fluorophore F ou respectivement d'un groupement inhibiteur de la fluorescence du groupement fluorophore F.

2. Substrat selon la revendication 1, **caractérisé par le fait que** les unités saccharidiques du squelette S sont choisies parmi le galactose, le mannose, le idose, le talose, le rhamnose, le glucose, le ribose, le fucose et leurs dérivés aminés ou acides.

3. Substrat selon la revendication 2, **caractérisé par le fait que** les dérivés aminés et acides des unités saccharidiques sont choisis parmi la galactosamine, la glucosamine, la lactosamine, l'acide glucuronique, l'acide iduronique et l'acide sialique.

4. Substrat selon la revendication 2 ou 3, **caractérisé par le fait que** les unités saccharidiques du squelette S des substrats de formule (I) sont choisies parmi la glucosamine, le galactose et l'acide glucuronique.

5. Substrat selon l'une quelconque des revendications précédentes, **caractérisé par le fait que** le squelette S est un oligosaccharide choisi parmi les oligosaccharides comportant de 4 à 9 unités saccharidiques.

6. Substrat selon l'une quelconque des revendications précédentes, **caractérisé par le fait que** Fonc est choisie parmi les fonctions amine primaire et thiol.

7. Substrat selon l'une quelconque des revendications précédentes, **caractérisé par le fait que** les sous-unités de formule (II) sont choisies parmi celles dans lesquelles Fonc est située en position ortho de l'atome de carbone porteur de l'atome d'oxygène.

8. Substrat selon l'une quelconque des revendications précédentes, **caractérisé par le fait que** les sous-unités de formule (III) sont choisies parmi celles dans lesquelles :
- X est un atome d'oxygène et Fonc une fonction amine primaire ou thiol,
- X est un atome d'azote et Fonc est une fonction amine primaire ou thiol.

9. Substrat selon l'une quelconque des revendications précédentes, **caractérisé par le fait que** les sous-unités de formule (IV) sont choisies parmi celles dans lesquelles R₁ est situé en position ortho de l'atome de carbone porteur de l'atome d'oxygène.

10. Substrat selon l'une quelconque des revendications précédentes, **caractérisé par le fait que** les sous-unités de formule (V) sont choisies parmi celles dans lesquelles :
- R₂ est un radical méthyle, m = 1, Fonc est une fonction amine primaire et Y est un atome d'oxygène,
- R₂ est un radical méthyle, m = 1, Fonc est une fonction amine primaire et Y = NH.

11. Substrat selon l'une quelconque des revendications précédentes, **caractérisé par le fait que** les sous-unités de formule (VI) sont choisies parmi celles dans lesquelles :
- W est un atome d'oxygène, n = 1, Fonc est une fonction amine primaire et Y est un atome d'oxygène,
- W représente NH, n = 1, Fonc est une fonction amine primaire et Y est un atome d'oxygène,
- W est un atome de soufre, n = 1, Fonc est une fonction amine primaire et Y est un atome d'oxygène.

12. Substrat selon l'une quelconque des revendications précédentes, **caractérisé par le fait qu'**il est choisi parmi les composés de formules (I-1) et (1-2) suivantes : 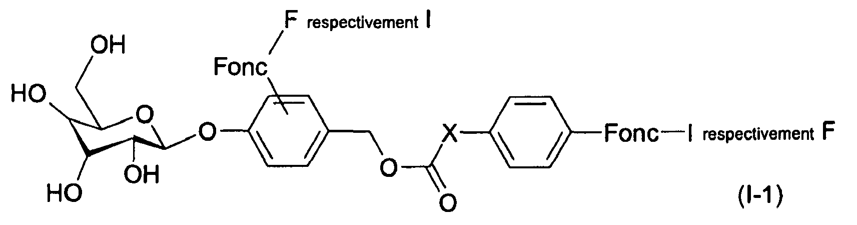 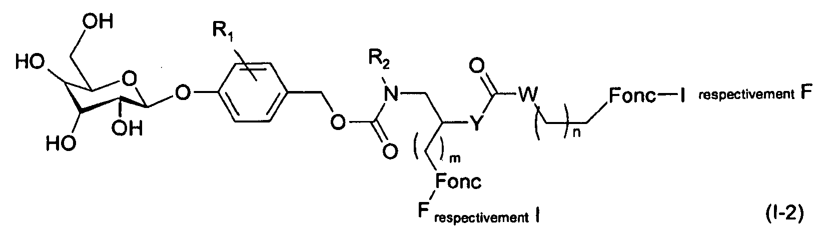 dans lesquelles, F, I, Fonc, X, Y, W, R₁ R₂, m et n ont les mêmes significations que celles indiquées par les revendications 1 à 11.

13. Substrat selon l'une quelconque des revendications précédentes, **caractérisé par le fait que** les groupements fluorophores F, sont choisis parmi la fluorescéine et ses dérivés ; les colorants fluorescents absorbant et émettant dans le proche infrarouge ; les cyanines fluorescentes ; le 7-hydroxy-9H-(1,3-dichloro-9,9-diméthylacridin-2-one) ; la rhodamine et ses dérivés ; les colorants fluorescents à amines réactives ; les difluorures de bore de dipyrrométhène ; les porphyrines ; les cyanines ; les oxazines ; les fluorophores dérivés du pyrène ; les dérivés diazoïques ; les dérivés du dansyle ; l'éosine ; l'érythrosine et les dérivés de sulforhodamine ; et les nanoparticules fluorescentes.

14. Substrat selon la revendication 13, **caractérisé par le fait que** le groupement F est choisi parmi les groupements fluorophores F absorbant et émettant dans le proche infrarouge.

15. Substrat selon l'une quelconque des revendications précédentes, **caractérisé par le fait que** le groupement I est un groupement fluorescent choisi parmi les groupements dont la fluorescence inhibe celle du groupement F.

16. Substrat selon la revendication 15, **caractérisé par le fait que** le groupement I est identique au groupement F.

17. Substrat selon l'une quelconque des revendications 1-14, **caractérisé par le fait que** le groupement I est un groupement fluorescent, différent du groupement F, et qui absorbe la fluorescence du groupement F par transfert d'énergie par résonance de fluorescence.

18. Substrat selon l'une quelconque des revendications précédentes, **caractérisé par le fait qu'**il est choisi parmi les composés de formule (I) dans lesquels :
i) le squelette S est une galactosamine, le bras espaceur est constitué d'une sous-unité B1 de formule (II) et d'une sous-unité B2 de formule (III) et F et I sont identiques ;
ii) le squelette S est une galactosamine, le bras espaceur est constitué d'une sous-unité B1 de formule (IV), d'une sous-unité B2 de formule (V) et d'une sous-unité B3 de formule (VI) et F et I sont identiques ;
iii) le squelette S est une galactosamine, le bras espaceur est constitué d'une sous-unité B1 de formule (IV), et d'une sous-unité B2 de formule (III) et F et I sont différents.

19. Substrat selon l'une quelconque des revendications 12 à 17, **caractérisé par le fait que** les substrats enzymatiques de formule (I-1) sont choisis parmi les composés suivants :

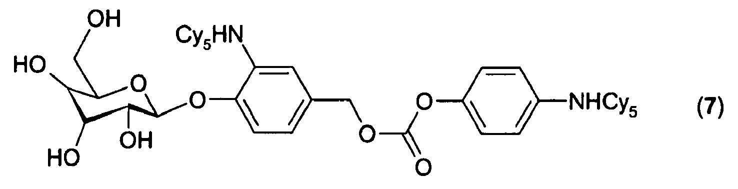

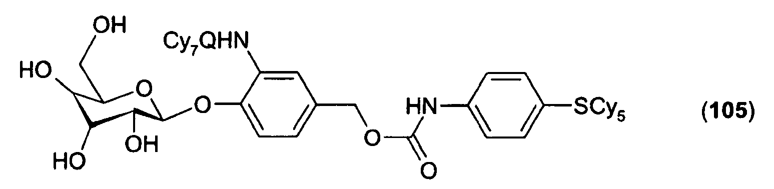

20. Substrat selon l'une quelconque des revendications 12 à 17, **caractérisé par le fait que** les substrats enzymatiques de formule (1-2) répondent à la formule suivante :

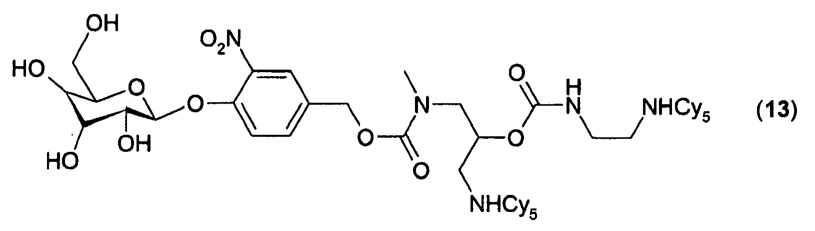

21. Utilisation d'au moins un substrat enzymatique de formule (I) tel que défini à l'une quelconque des revendications précédentes, à titre de réactif fluorescent pour la détection d'une activité enzymatique *in vitro.*

22. Utilisation d'au moins un substrat enzymatique de formule (I) tel que défini à l'une quelconque des revendications 1 à 20 et dans lequel le groupement fluorophore F est choisi parmi les groupements absorbant et émettant dans le proche infrarouge, pour la préparation d'un réactif de diagnostic destiné à l'imagerie fonctionnelle *in vivo.*

23. Utilisation selon la revendication 22, **caractérisée par le fait que** ledit réactif est destiné à imager, par fluorescence, l'expression des gènes rapporteurs LacZ et gusA de *E. coli.*

24. Réactif de diagnostic, **caractérisé par le fait qu'**il comprend au moins une solution constituée d'eau ou d'un mélange d'eau et d'au moins un solvant organique, ladite solution renfermant au moins un substrat enzymatique de formule (I) tel que défini à l'une quelconque des revendications 1 à 20.

25. Réactif selon la revendication 24, **caractérisé par le fait qu'**il s'agit d'un réactif de diagnostic *in vivo* et que le substrat enzymatique de formule (I) comprend au moins un groupement fluorophore F choisi parmi les groupements fluorophores absorbant et émettant dans le proche infrarouge.

26. Réactif selon la revendication 24 ou 25, **caractérisé par le fait que** la concentration du ou des substrats enzymatiques de formule (I) est comprise entre 1 µM et 1 mM.

## Patentansprüche

1. Fluoreszierendes Enzymsubstrat, **dadurch gekennzeichnet, dass** es der folgenden Struktur (I) entspricht:
S-B(I)ₙ-F (I)
wobei:
- S ein Gerüst mit Saccharidnatur ist, das aus wenigstens einer Saccharideinheit besteht und ausgewählt ist aus Monosacchariden, Oligosacchariden mit 2 bis 9 Saccharideinheiten und Polysacchariden mit wenigstens 10 Saccharideinheiten;
- F eine Fluorophorgruppe ist, die von dem Spacerarm B getragen wird;
- I eine Inhibitorgruppe der Fluoreszenz von F ist; wobei die Gruppe I ein seitlicher Substituent wenigstens einer Untereinheit des Spacerarms B ist;
- mit der Maßgabe, dass keine kovalente Bindung die Fluorophorgruppe direkt mit der Inhibitorgruppe verbindet;
- n eine ganze Zahl gleich 1 oder 2 ist;
- B einen selbst-abspaltbaren Spacerarm darstellt, der aus einer oder mehreren Untereinheiten besteht; wobei der Arm B in einer anomeren Position der Saccharideinheit S gebunden ist, wobei die anomere Bindung unabhängig in α- oder β-Position ist, wobei der Arm B einer Stelle entspricht, die durch ein Enzym erkannt und gespalten wird, und eine Untereinheit B1 umfasst, die ausgewählt ist aus:
i) aromatischen monocyclischen Gruppen der folgenden Formel (II) : 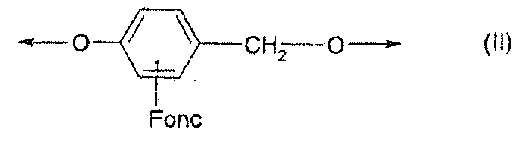 in der:
- Fonc eine chemische Funktion ist, die gegenüber einer komplementären chemischen Funktion einer Fluorophorgruppe F bzw. einer Inhibitorgruppe für die Fluoreszenz einer Fluorophorgruppe F reaktiv ist,
- der Pfeil, der vom Sauerstoffatom, das direkt von dem Phenylring getragen wird, ausgeht, den Verknüpfungspunkt der Untereinheit B1 mit einer Saccharideinheit des Spacerarms über eine kovalente Bindung mit dem Kohlenstoffatom, das sich in der anomeren Position 1 der Saccharideinheit befindet, darstellt,
- der Pfeil, der von dem Sauerstoffatom, das an den Rest -CH₂- gebunden ist, ausgeht, den Verknüpfungspunkt der Untereinheit B1 mit einer Untereinheit B2 darstellt, welche ausgewählt ist aus den aromatischen Gruppen der folgenden Formel (III) : 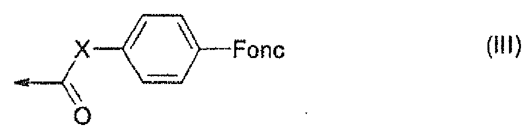 in der:
• Fonc eine chemische Funktion ist, die gegenüber einer komplementären chemischen Funktion einer Inhibitorgruppe der Fluoreszenz der Gruppe F bzw. einer Fluorophorgruppe F reaktiv ist;
• der Pfeil den Verknüpfungspunkt der Untereinheit B2 mit der Untereinheit B1 über eine kovalente Bindung mit dem Sauerstoffatom der Untereinheit B1 darstellt,
• X für O, NH oder S steht, und
ii) den monocyclischen aromatischen Gruppen der folgenden Formel (IV): 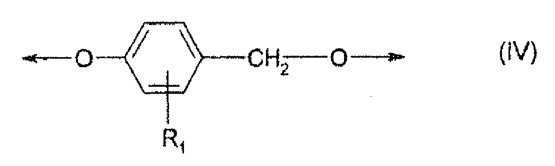 in der:
- R₁ ausgewählt ist aus Nitro-, Sulfat-, Amingruppen und einer mit einer Schutzgruppe geschützten Amingruppe,
- der Pfeil, der von dem Sauerstoffatom, das direkt von dem Kohlenstoffatom des Phenylrings getragen wird, ausgeht, den Verknüpfungspunkt der Untereinheit B1 mit einer Saccharideinheit des Spacerarms über eine kovalente Bindung mit dem Kohlenstoffatom, das sich in der anomeren Position 1 der Saccharideinheit befindet, darstellt,
- der Pfeil, der von dem Sauerstoffatom, das an den Rest -CH₂- gebunden ist, ausgeht, den Verknüpfungspunkt der Untereinheit B1 mit einer Untereinheit B2 darstellt, welche aus Gruppen der folgenden Formel (V) ausgewählt ist: 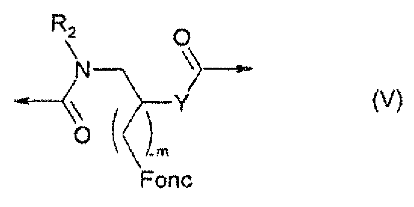 in der:
• R₂ ein Wasserstoffatom oder ein C₁-C₄-Alkylrest ist,
• Fonc eine chemische Funktion ist, die gegenüber einer komplementären chemischen Funktion einer Fluorophorgruppe F bzw. einer Inhibitorgruppe der Fluoreszenz einer Fluorophorgruppe F reaktiv ist;
• n eine ganze Zahl von 1 bis 10 ist;
• Y für 0, NH oder S steht;
• die Pfeile den Verknüpfungspunkt der Untereinheit B2 einerseits mit der Untereinheit B1 und andererseits mit einem Stickstoff- oder Sauerstoffatom, das von einer Untereinheit B3 der folgenden Formel (VI) getragen wird, darstellen: 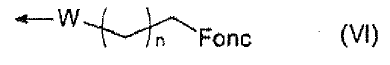 in der:
• W für O, NH oder S steht;
• der Pfeil den Verknüpfungspunkt des Stickstoff-, Schwefel-, oder Sauerstoffatoms, das durch W bezeichnet wird, über eine kovalente Bindung mit einem Kohlenstoffatom der Untereinheit B2 darstellt,
• n eine ganze Zahl von 1 bis 10 ist;
• Fonc eine chemische Funktion ist, die gegenüber einer komplementären chemischen Funktion einer Fluorophorgruppe F bzw. einer Inhibitorgruppe der Fluoreszenz der Fluorophorgruppe F reaktiv ist.

2. Substrat nach Anspruch 1, **dadurch gekennzeichnet, dass** die Saccharideinheiten des Gerüsts S ausgewählt sind aus Galactose, Mannose, Idose, Talose, Rhamnose, Glucose, Ribose, Fucose und deren Amin- oder Säurederivaten.

3. Substrat nach Anspruch 2, **dadurch gekennzeichnet, dass** die Amin- und Säurederivate der Saccharideinheiten aus Galactosamin, Glucosamin, Lactosamin, Glucoronsäure, Idoronsäure und Sialinsäure ausgewählt sind.

4. Substrat nach Anspruch 2 oder 3, **dadurch gekennzeichnet, dass** die Saccharideinheiten des Gerüsts S der Substrate der Struktur (I) ausgewählt sind aus Glucosamin, Galactose und Glucoronsäure.

5. Substrat nach einem der vorhergehenden Ansprüche, **dadurch gekennzeichnet, dass** das Gerüst S ein Oligosaccharid, ausgewählt aus Oligosacchariden, die 4 bis 9 Saccha-rideinheiten umfassen, ist.

6. Substrat nach einem der vorhergehenden Ansprüche, **dadurch gekennzeichnet, dass** Fonc aus primären Amin- und Thiolfunktionen ausgewählt ist.

7. Substrat nach einem der vorhergehenden Ansprüche, **dadurch gekennzeichnet, dass** die Untereinheiten der Formel (II) aus denjenigen ausgewählt sind, in denen Fonc sich in ortho-Position zu dem Kohlenstoffatom, das Träger des Sauerstoffatoms ist, befindet.

8. Substrat nach einem der vorhergehenden Ansprüche, **dadurch gekennzeichnet, dass** die Untereinheiten der Formel (III) ausgewählt sind, aus denjenigen, in denen:
- X ein Sauerstoffatom ist und Fonc eine primäre Amin- oder Thiolfunktion ist,
- X ein Stickstoffatom ist und Fonc eine primäre Amin- oder Thiolfunktion ist.

9. Substrat nach einem der vorhergehenden Ansprüche, **dadurch gekennzeichnet, dass** die Untereinheiten der Formel (IV) aus denjenigen ausgewählt sind, in denen R₁ sich in Ortho-Position zu dem Kohlenstoffatom, das Träger des Sauerstoffatoms ist, befindet.

10. Substrat nach einem der vorhergehenden Ansprüche, **dadurch gekennzeichnet, dass** die Untereinheiten der Formel (V) ausgewählt sind aus denjenigen, in denen:
- R₂ ein Methylrest ist, m gleich 1 ist, Fonc eine primäre Aminfunktion ist und Y ein Sauerstoffatom ist,
- R₂ ein Methylrest ist, m gleich 1 ist, Fonc eine primäre Aminfunktion ist und Y gleich NH ist.

11. Substrat nach einem der vorhergehenden Ansprüche, **dadurch gekennzeichnet, dass** die Untereinheiten der Formel (VI) ausgewählt sind aus denjenigen, in denen:
- W ein Sauerstoffatom ist, n gleich 1 ist, Fonc eine primäre Aminfunktion ist und Y ein Sauerstoffatom ist,
- W für NH steht, n gleich 1 ist, Fonc eine primäre Aminfunktion ist und Y ein Sauerstoffatom ist,
- W ein Schwefelatom ist, n gleich 1 ist, Fonc eine primäre Aminfunktion ist und Y ein Sauerstoffatom ist.

12. Substrat nach einem der vorhergehenden Ansprüche, **dadurch gekennzeichnet, dass** es aus den Verbindungen der folgenden Strukturen (I-1) und (I-2) ausgewählt ist: 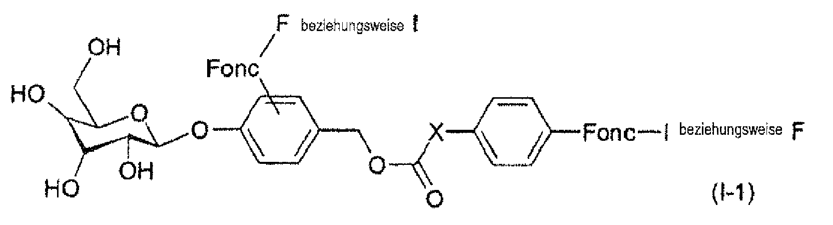 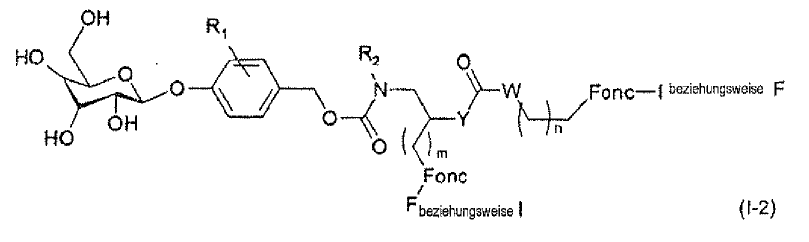 in denen F, I, Fonc, X, Y, W, R₁, R₂, m und n die selben Bedeutungen, wie die, die für die Ansprüche 1 bis 11 angegeben sind, haben.

13. Substrat nach einem der vorhergehenden Ansprüche, **dadurch gekennzeichnet, dass** die Fluorophor-Gruppen F ausgewählt sind aus Fluorescein und seinen Derivaten; Fluoreszenzfarbstoffen, die im nahen Infrarotbereich absorbieren und emittieren; fluoreszierenden Cyaninen; 7-Hydroxy-9*H*-(1,3-dichlor-9,9-dimethylacridin-2-on), Rhodamin und seinen Derivaten; fluoreszenzfarbstoffen, die reaktive Amine umfassen; Dipyrromethenbordifluorid; Pophyrinen; Cyaninen; Oxazinen; Fluorophoren, die von Pyren abgeleitet sind; Diazoderivaten; Dansylderivaten; Eosin; Erythrosin und Sulforhodaminderivaten und fluoreszierenden Nanopartikeln.

14. Substrat nach Anspruch 13, **dadurch gekennzeichnet, dass** die Gruppe F ausgewählt ist aus Fluorophorgruppen F, die im nahen Infrarotbereich absorbieren und emittieren.

15. Substrat nach einem der vorhergehenden Ansprüche, **dadurch gekennzeichnet, dass** die Gruppe I eine fluoreszierende Gruppe ist, die aus Gruppen ausgewählt ist, deren Fluoreszenz diejenige der Gruppe F inhibiert.

16. Substrat nach Anspruch 15, **dadurch gekennzeichnet, dass** die Gruppe I mit der Gruppe F identisch ist.

17. Substrat nach einem der Ansprüche 1-14, **dadurch gekennzeichnet, dass** die Gruppe I eine fluoreszierende Gruppe ist, die sich von der Gruppe F unterscheidet, und die die Fluoreszenz der Gruppe F durch Fluoreszenzresonanz-Energietransfer absorbiert.

18. Substrat nach einem der vorhergehenden Ansprüche, **dadurch gekennzeichnet, dass** es ausgewählt ist aus Verbindungen der Struktur (I), in denen:
i) das Gerüst S ein Galactosamin ist, der Spacerarm aus einer Untereinheit B1 der Formel (II) und einer Untereinheit B2 der Formel (III) besteht und F und I identisch sind;
ii) das Gerüst S ein Galactosamin ist, der Spacerarm aus einer Untereinheit B1 der Formel (IV), einer Untereinheit B2 der Formel (V) und einer Untereinheit B3 der Formel (VI) besteht, und F und I identisch sind;
iii) das Gerüst S ein Galactosamin ist, der Spacerarm aus einer Untereinheit B1 der Formel (IV) und einer Untereinheit B2der Formel (III) besteht und F und I unterschiedlich sind.

19. Substrat nach einem der Ansprüche 12 bis 17, **dadurch gekennzeichnet, dass** die Enzymsubstrate der Struktur (I-1) aus den folgenden Verbindungen ausgewählt sind:

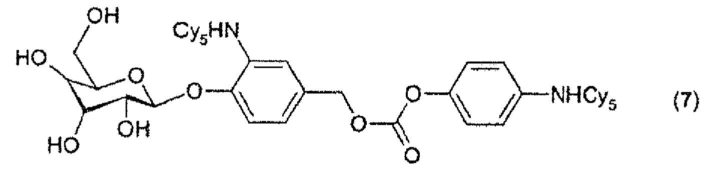

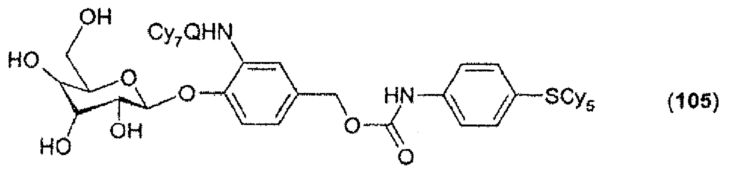

20. Substrat nach einem der Ansprüche 12 bis 17, **dadurch gekennzeichnet, dass** die Enzymsubstrate der Struktur (1-2) der folgenden Formel entsprechen:

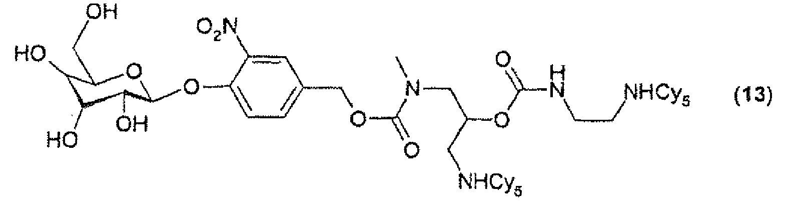

21. Verwendung wenigstens eines Enzymsubstrats der Struktur (I), wie es in einem der vorhergehenden Ansprüche definiert ist, als fluoreszierendes Reagens zum Nachweis einer Enzymaktivität *in vitro*.

22. Verwendung wenigstens eines Enzymsubstrats der Struktur (I), wie es in einem der Ansprüche 1-20 definiert ist, in dem die Fluorophorgruppe F aus den Gruppen, die im nahen Infrarotbereich absorbieren und emittieren, ausgewählt ist, zur Herstellung eines Diagnosereaganzes, das zur funktionellen Bildgebung *in vivo* bestimmt ist.

23. Verwendung nach Anspruch 22, **dadurch gekennzeichnet, dass** das Reagens zur Abbildung durch Fluoreszenz der Expression der E. coli-, LacZ- und gusA-Reportergene bestimmt ist.

24. Diagnosereagens, **dadurch gekennzeichnet, dass** es wenigstens eine Lösung umfasst, die aus Wasser oder einem Gemisch aus Wasser und wenigstens einem organischen Lösungsmittel besteht, wobei die Lösung wenigstens ein Enzymsubstrat der Struktur (I), wie es in einem der Ansprüche 1 bis 20 definiert ist, umfasst.

25. Reagens nach Anspruch 24, **dadurch gekennzeichnet, dass** es ein *in-vivo*-Diagnosereagens ist und, dass das Enzymsubstrat der Struktur (I) wenigstens eine Fluorophorgruppe F umfasst, die aus Fluorophorgruppen ausgewählt ist, die im nahen Infrarotbereich absorbieren und emittieren.

26. Reagens nach Anspruch 24 oder 25, **dadurch gekennzeichnet, dass** die Konzentration des oder der Enzymsubstrate der Struktur (I) zwischen 1 µM und 1 mM liegt.

## Claims

1. Fluorescent enzymatic substrate, **characterised by** the fact that it has the following structure (I):
S-B(I)ₙ-F (I)
in which:
- S is a backbone of a saccharidic nature which is formed by at least one saccharide unit and which is selected from the monosaccharides, the oligosaccharides having from 2 to 9 saccharide units and the polysaccharides having at least 10 saccharide units:
- F is a fluorophore group carried by the spacer arm B,
- I is a group which quenches the fluorescence of F, the said group I being a lateral substituent of at least one sub-unit of the spacer arm B,
it being understood that there is no covalent bond directly linking the fluorophore group to the quenching group
- n is an integer equal to 1 or 2,
- B is a spontaneously cleavable spacer arm formed by one or more sub-units, the said arm B being fixed in an anomeric position of the said saccharide unit S, the anomeric bond being either in the α or β position, the said arm B corresponding to a site which is recognised and cleaved by an enzyme and which comprises a sub-unit B1 selected from:
i) the monocyclic aromatic groups having the following general formula (II): 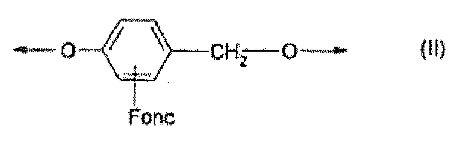 in which:
- Fonc is a functional chemical group which is reactive with a functional chemical group complementary, in respective cases, to a fluorophore group F or to a group quenching the fluorescence of a fluorophore group F,
- the arrow starting from the oxygen atom carried directly by the phenyl ring represents the point at which the said sub-unit B1 is attached to a saccharide unit of the spacer arm by a covalent bond to the carbon atom situated in anomeric position 1 of the said saccharide unit,
- the arrow starting from the oxygen atom bonded to the radical -CH₂- represents the point at which the said sub-unit B1 is attached to a sub-unit B2 selected from the aromatic groups having the following general formula (III): 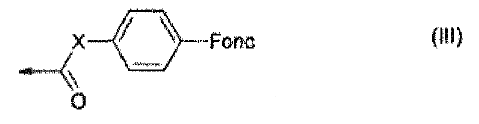 in which:
• Fonc is a functional chemical group which is reactive with a functional chemical group complementary, in respective cases, to a group quenching the fluorescence of the group F or to a fluorophore group F,
• the arrow represents the point at which the said sub-unit B2 is attached to the sub-unit B1 by a covalent bond to the oxygen atom of the sub-unit B1, and
• X is O, NH or S,
ii) the monocyclic aromatic groups having the following general formula (IV): 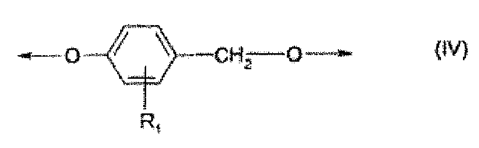 in which:
- R₁ is selected from the following groups: nitro, sulphate, amine, or amine protected by a protective group,
- the arrow starting from the oxygen atom carried directly by the carbon atom of the phenyl ring represents the point at which the said sub-unit B1 is attached to a saccharide unit of the spacer arm by a covalent bond to the carbon atom situated in anomeric position 1 of the said saccharide unit,
- the arrow starting from the oxygen atom bonded to the radical -CH₂- represents the point at which the said sub-unit B1 is attached to a sub-unit B2 selected from the groups having the following general formula (V): 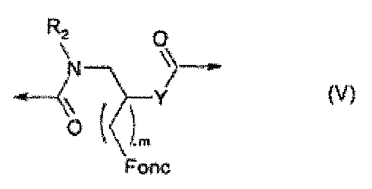 in which:
• R₂ is a hydrogen atom or a C₁-C₄ alkyl radical,
• Fonc is a functional chemical group which is reactive with a functional chemical group complementary, in respective cases, to a fluorophore group F or to a group quenching the fluorescence of a fluorophore group F,
• m is an integer ranging from 1 to 10,
• Y is O, NH or S,
• the arrows represent the points at which the said sub-unit B2 is attached on the one hand to the sub-unit B1 and on the other hand to a nitrogen or oxygen atom which is carried by a sub-unit B3 having the following general formula (VI) : 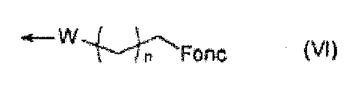 in which:
• W is 0, NH or S,
• the arrow represents the point at which the nitrogen, sulphur or oxygen atom indicated by W is attached by a covalent bond to a carbon atom of the sub-unit B2,
• n is an integer ranging from 1 to 10,
• Fonc is a functional chemical group which is reactive with a functional chemical group complementary, in respective cases, to a fluorophore group F or to a group quenching the fluorescence of a fluorophore group F,

2. Substrate according to claim 1, **characterised by** the fact that the saccharide units of the backbone S are selected from galactose, mannose, idose, talose, rhamnose, glucose, ribose, fucose, and the amino or acid derivatives thereof.

3. Substrate according to claim 2, **characterised by** the fact that the amino and acid derivatives of the saccharide units are selected from galactosamine, glucosamine, lactosamine, glucuronic acid, iduronic acid and sialic acid.

4. Substrate according to claim 2 or 3, **characterised by** the fact that the saccharide units of the backbone S of the substrates having the general formula (I) are selected from glucosamine, galactose and glucuronic acid.

5. Substrate according to any one of the preceding claims, **characterised by** the fact that the backbone S is an oligoisaccharide selected from the oligoisaccharides comprising from 4 to 9 saccharide units.

6. Substrate according to any one of the preceding claims, **characterised by** the fact that Fonc is selected from the primary amine and the thiol functional groups.

7. Substrate according to any one of the preceding claims, **characterised by** the fact that the sub-units having the general formula (II) are selected from those in which the functional group Fonc is situated in the ortho position of the carbon atom carrying the oxygen atom.

8. Substrate according to any one of the preceding claims, **characterised by** the fact that the sub-units having the general formula (III) are selected from those in which:
- X is an oxygen atom and Fonc is a primary amine functional group or a thiol functional group, or
- X is a nitrogen atom and Fonc is a primary amine functional group or a thiol functional group.

9. Substrate according to any one of the preceding claims, **characterised by** the fact that the sub-units having the general formula (IV) are selected from those in which R₁ is situated in the ortho position of the carbon atom carrying the oxygen atom.

10. Substrate according to any one of the preceding claims, **characterised by** the fact that the sub-units having the general formula (V) are selected from those in which
- R₂ is a methyl radical, m = 1, Fonc is a primary amine functional group and Y is an oxygen atom, or
- R₂ is a methyl radical, m = 1, Fonc is a primary amine functional group and Y = NH.

11. Substrate according to any one of the preceding claims, **characterised by** the fact that that the sub-units having the general formula (VI) are selected from those in which
- W is an oxygen atom, n = 1, Fonc is a primary amine functional group and Y is an oxygen atom,
- W is NH, n = 1, Fonc is a primary amine functional group and Y is an oxygen atom, or
- W is a sulphur atom, n = 1, Fonc is a primary amine functional group and Y is an oxygen atom.

12. Substrate according to any one of the preceding claims, **characterised by** the fact that it is selected from the compounds having the following general formulas (I-1) and (I-2) 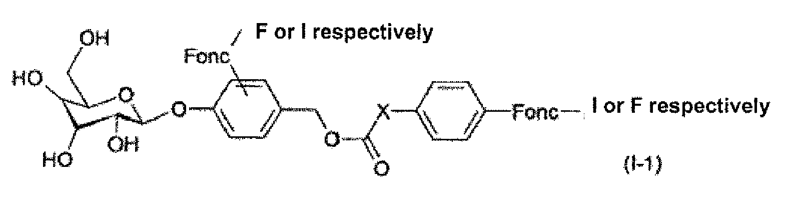 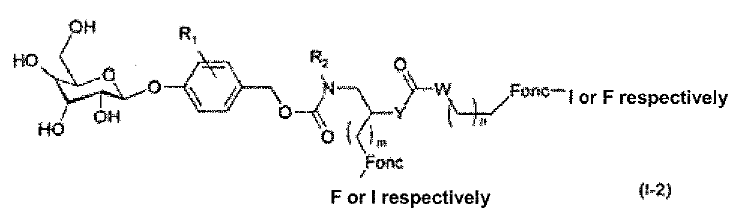 in which F, I, Fonc, X, Y, W, R₁, R₂, m and n have the same meanings as those indicated in claims 1 to 11.

13. Substrate according to any one of the preceding claims, **characterised by** the fact that the fluorophore groups F are selected from fluorosceine and the derivatives thereof; the fluorescent dyes which absorb and emit in the near infrared; the fluorescent cyanines; 7-hydroxy-9H-(1,3-dichloro-9,9-dimethylacridin-2-one); rhodamine and the derivatives thereof; the amine-reactive fluorescent dyes; the dipyrromethene boron difluorides; the porphyrines; the cyanines; the oxazines; the fluorophores derived from pyrene; the diazoic derivatives; the dansyle derivatives; eosine; erythrosine and the derivatives of sulphorhodamine; and fluorescent nanoparticles.

14. Substrate according to claim 13, **characterised by** the fact that the group F is selected from the fluorophore groups F which absorb and emit in the near infrared.

15. Substrate according to any one of the preceding claims, **characterised by** the fact that the group I is a fluorescent group which is selected from the groups whose fluorescence quenches that of the group F.

16. Substrate according to claim 15, **characterised by** the fact that the group I is the same as the group F.

17. Substrate according to any one of claims 1 - 14, **characterised by** the fact that the group I is a fluorescent group different from the group F which absorbs the fluorescence of group F by fluorescence resonance energy transfer.

18. Substrate according to any one of the preceding claims, **characterised by** the fact that it is selected from the compounds having the general formula (I) in which:
i) the backbone S is a galactosamine, the spacer arm is formed by a sub-unit B1 having the general formula (II) and by a sub-unit B2 having the general formula (III), and F and I are the same,
ii) the backbone S is a galactosamine, the spacer arm is formed by a sub-unit B1 having the general formula (IV) and by a sub-unit B2 having the general formula (V) and by a sub-unit B3 having the general formula (VI), and F and I are the same, or
iii) the backbone S is a galactosamine, the spacer arm is formed by a sub-unit B1 having the general formula (IV) and by a sub-unit B2 having the general formula (III), and F and I are different.

19. Substrate according to any one of claims 12 to 17, **characterised by** the fact that the enzymatic substrates having the general formula (I-1) are selected from the following compounds:

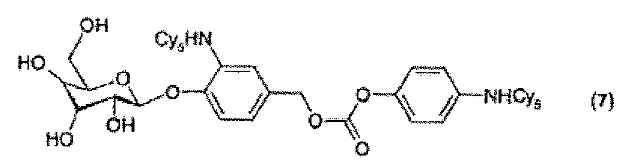

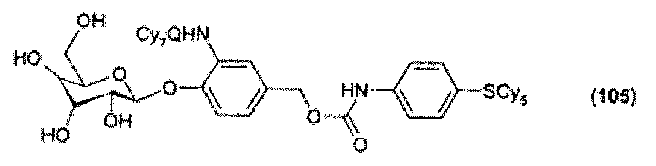

20. Substrate according to any one of claims 12 to 17, **characterised by** the fact that the enzymatic substrates having the general formula (1-2) have the following formula:

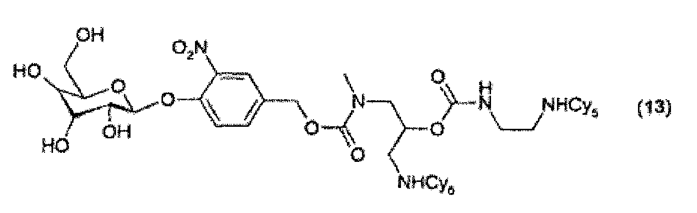

21. Use of at least one enzymatic substrate having the general formula (I), as defined in any one of the preceding claims, as a fluorescent reagent for the in vitro detection of enzymatic activity.

22. Use of at least one enzymatic substrate having the general formula (I), as defined in any one of claims 1 to 20, in which the fluorophore group F is selected from the groups which absorb and emit in the near infrared, for preparing a diagnostic reagent intended for functional in vivo imaging.

23. Use according to claim 22, **characterised by** the fact that the said reagent is intended to produce images, by fluorescence, of the expression of the E. coli LacZ and gusA reporter genes.

24. Diagnostic reagent, **characterised by** the fact that it comprises at least one solution formed by water or a mixture of water and at least one organic solvent, the said solution containing at least one enzymatic substrate having the general formula (I), as defined in any one of claims 1 to 20.

25. Reagent according to claim 24, **characterised by** the fact that it is a reagent for in vivo diagnosis and that the enzymatic substrate having the general formula (I) comprises at least one fluorophore group F which is selected from the fluorophore groups which absorb and emit in the near infrared.

26. Reagent according to claim 24 or 25, **characterised by** the fact that the concentration of the enzymatic substrate or substrates having the general formula (I) is between 1 µM and 1 mM.
